# EUROPEAN PATENT APPLICATION

(11) **EP 0 955 054 A1**
(43) Date of publication of application: **10.11.1999**
(21) Application number: 97911479.0
(22) Date of filing: 07.11.1997
(51) Int. Cl.: A61K 38/06, A61K 38/55

(54) **AIDS REMEDY**

(30) Priority: 08.11.1996 JP 31277296; 09.12.1996 JP 34455096; 09.10.1997 JP 29336597
(71) Applicant: JAPAN ENERGY CORPORATION, Tokyo 105-0001 (JP)
(72) Inventor: SATO, Hideharu, Minato-ku, Tokyo 105 (JP); SHINTANI, Makoto, Minato-ku, Tokyo 105 (JP); FUKAZAWA, Tominaga, Japan Energy Corporation, Toda-shi, Saitama 335 (JP); MUTO, Akihiro, Japan Energy Corporation, Toda-shi, Saitama 335 (JP); TERAJIMA, Keisuke, Japan Energy Corporation, Toda-shi, Saitama 335 (JP)
(74) Representative: Wakerley, Helen Rachael
(86) International application number: JP9704057
(87) International publication number: WO9820888

(57) **Abstract**

A AIDS therapeutic composition comprising medicaments which possess an HIV protease inhibition activity which is designed so that each medicament can achieve a higher treatment effect. The composition comprises KNI-272 and one compound selected from the group consisting of Saquinavir, Ritonavir, Indinavir, and Nelfinavir, exhibiting as essential active components a human immunodeficiency virus protease inhibition action, in such an amount and proportion to exhibit a synergistic treatment effect.

## Description

The present invention relates to a drug composition used for treating acquired immune deficiency syndrome (AIDS) caused by a human immunodeficiency virus. More particularly, the present invention relates to an AIDS therapeutic composition which comprises two chemicals as essential components, each chemical exhibiting an inhibitive action against enzyme activity of a protease originating from this virus, wherein the synergistic treatment effects of the two chemicals, such as a synergistic increase in anti-virus activity, a synergistic increase in the peak value of blood concentration, a synergistic extension of the half-life of the blood concentration, are utilized by suitable combinations of the proportion and dose of these two chemicals.

### BACKGROUND OF THE INVENTION

The human immunodeficiency virus (HIV) which causes acquired immune deficiency syndrome (AIDS) produces Gag proteins and reverse transcription enzymes used for formation of the said virus particles as precursor proteins prior to a series of polypeptides in host cells. These polypeptide precursor proteins can exhibit their functions only when cut into specific sizes by a protease (HIV protease) originating from said virus. For this reason, an HIV protease inhibitor becomes a compound exhibiting an anti-virus activity by inhibiting the HIV protease enzyme activity and by blocking formation and maturation of infectious virus particles. Several HIV protease inhibitors have already been reported. One of these is a compound similar to a synthetic peptide and called a transition-state mimetic (see T. Robins, J. Plattner, J. Acquir. Immun. Defic. Syndr., 6, 162 (1993), etc.).

Several compounds have been reported heretofore as useful HIV protease inhibitors. Such compounds include, for example, a hydroxyethylamine-type derivative such as Ro31-8959 (e.g. N. A. Roberts et al., Science 248, 358-361 (1990)) containing a phenylalanine φ [CH(OH)CH₂N] decahydroisoquinoline carboxylic acid bone which is similar to the amino acid sequence, -Tyr···Pro- or -Phe···Pro-, selectively cut by an HIV protease, and a hydroxymethylcarboxamide-type derivative such as a peptide derivative including norstatine bone, such as a phenylalanine φ [CH(OH)C(O)N] proline (e.g. T. F. Tam et al., J. Med. Chem. 35, 1318-1320 (1992)). The present inventors have also discovered that a group of synthetic peptide compounds which are transition state mimetics including a 3-amino-2-hydroxy-4-phenylbutanoic acid residue exhibit a strong HIV protease inhibitive action and are useful as anti-AIDS medicines. The inventors have filed a patent application relating to the HIV protease inhibitor (Japanese Patent Application Laid-open No. 170722/1993).

These transition state mimetics are expected to become next generation anti-AIDS medicines of great promise succeeding nucleic acid derivative-type reverse transcription enzyme inhibitors such as AZT (azidothymidine), DDC (dideoxycytidine), and DDI (dideoxyinosine) which are already clinically used as anti-AIDS drugs. Clinical tests and research on these drugs are ongoing. Specifically, studies relating to clinical application of these transition state mimetics as anti-AIDS medicine are being actively undertaken, wherein the HIV protease inhibitive action to control formation of said virus particles in host cells and thereby to inhibit proliferation and infection of HIV is utilized (see, for example, Nakajima et al, Monthly Medicines, Vol. 35, 2983-2989 (1993)) . The medicine is currently available commercially. This anti-AIDS medicine containing the HIV protease inhibitor as an active ingredient is administered to HIV-infected subjects in particular to prevent the subjects from becoming sick with AIDS, to ease symptoms of AIDS, to control progress of the symptoms, and to prevent the AIDS symptoms from worsening.

In addition, the combined use of a transition state mimetic exhibiting an HIV protease inhibitive action and a nucleic acid-based reverse transcription enzyme inhibitor is reported to provide a synergistic anti-virus effect of both substances which have completely different action mechanisms. Also, with regard to the nucleic acid derivative-type reverse transcription enzyme inhibitors themselves there is a report describing a synergistic treatment effect obtained when several different kinds of these enzyme inhibitors are administered in combination. Specifically, these nucleic acid analogues are replaced with natural nucleic acids when DNA is produced from an RNA gene of a virus by reverse transcription, making it impossible for the DNA chain to further extend, which results in interruption of the reverse transcription. The nucleic acid analogues replaced respectively by different natural nucleic acid groups are therefore capable of exhibiting synergistic effects. On the other hand, the transition state mimetic substances which cause an inhibitive action by selectively and irreversibly bonding to an enzymatic active site on the HIV protease replacing its substrate peptide have been considered to exhibit no synergistic effects because these substances have an identical active site. Because of this conventionally established thinking a combined use of two or more of these peptide-like compounds which are HIV protease inhibitors has not been tried.

Development of an AIDS therapeutic composition comprising a transition state mimetic possessing an HIV protease inhibition effect has been focused on development of a novel compound having a superior enzymatic inhibitive capability, and greater concern has been directed to studies relating to preparation formulas which improve oral absorptivity of known peptide-like compounds. These studies have achieved certain results in bringing some AIDS therapeutic composition comprising an HIV protease inhibitor as an active ingredient into practical use. However, some undesirable adverse effects caused by the requirement of maintaining a certain blood concentration level are pointed out, relating to a drug using a single compound. Development of an improved method of administration and a preparation formula which can achieve the same or more treatment effect at a lower dose have therefore been desired.

For instance, development of combined use of two or more kinds of HIV protease inhibitor each possessing theoretically the same active site but capable of bringing about a synergistic treatment effect as opposed to the above-mentioned conventional anticipation, specifically, development of a combination of medicines or a dosing method which increases the synergistic anti-virus activity, is desired. Also desired is development of a combination of medicines or a dosing method comprising use of two or more kinds of HIV protease inhibitors which exhibits a synergistic treatment effect, specifically, which enhances the maximum concentration in blood, extends residence time in blood, or controls the rate of excretion after oral administration, thereby synergistically increasing the period of time wherein the blood concentration is kept above the level at which these active ingredients can exhibit the desired treatment effect.

If such a synergistic treatment effect can be achieved by the combined use of the above-mentioned two or more kinds of HIV protease inhibitors, the dose of an individual medicine can be relatively decreased, whereby undesirable adverse effects which accompany the administration of a large amount of medicine can be mitigated. In practice, extension of the period of time during which the concentration in blood is kept above the level required for the medicine to be effective makes it possible to extend the intervals of administration. This greatly contributes to relaxing the mental and physical burdens of the patients.

In addition, various commercially available reverse transcription enzyme inhibitors of a nucleic acid analog type are reported to decrease the treatment effect if continuously administered to a patient for a long period of time. A study investigating the cause of this failure revealed that variation induced in a reverse transcription enzyme of HIV origin produces a variant virus which acquired drug resistance. Some commercially new available HIV protease inhibitors were also confirmed to induce variation in the HIV protease if continuously administered for a long period of time and to produce a variant virus with acquired drug resistance. However, there is no way to avoid appearance of the variant virus with acquired drug resistance if the drug is singly administered. At the present, a patient in whom such a variant virus with acquired drug resistance has once been discovered has no other option but to be provided with another medicine. Because appearance of such a variant virus with acquired drug resistance is accelerated by administration of a single drug at a large dosage, a method of administration wherein dosage of individual medicament is relatively limited to maintain a high anti-virus activity is desired.

The above-described method of administering two or more kinds of HIV protease inhibitor is thus expected also to achieve the improved synergistically treatment effect as a means for avoiding substantially such appearance of a variant virus with drug resistance acquired. In this respect, development of a combination of medicines or a dosing method comprising use of two or more drugs with a relatively reduced individual dose is desired.

### DISCLOSURE OF THE INVENTION

The present invention has been achieved to solve the above-mentioned problems and has an object of providing a method of dosing transition state mimetic substances possessing an HIV protease inhibitive action in such a manner that the substances can exhibit increased synergistic anti-virus activity or achieve synergistic extension of time during which an effective concentration in blood is maintained, and also providing a drug composition adapted for the dosing method. More specifically, the present invention relates to a method of administering two or more substances possessing an HIV protease inhibitive action as an active ingredient simultaneously in such a manner that the substances exhibit increased synergistic anti-virus activity, and to an AIDS therapeutic composition adapted for the dosing method. Furthermore, the present invention relates to a method of administering two or more substances possessing an HIV protease inhibitive action simultaneously in such a manner that each substance possessing an HIV protease inhibitive action can be maintained in an effective range of concentration in blood for a prolonged period of time and this extension of time occurs synergistically, and also to an AIDS therapeutic agent composition adapted for the dosing method.

To achieve these objectives, the present inventors have conducted extensive studies, using a number of HIV protease inhibitors which are known to possess high anti-virus activity, to investigate suitable combinations of these compounds which exhibit synergistic increase in oral absorptivity during oral administration or synergistic extension of the half-life period of concentration in blood. As a result of experiments undertaken based on this study, the present inventors have found that among various HIV protease inhibitors which are various peptide-like compounds certain combinations of specific HIV protease inhibitors exhibit a remarkable synergistic increase in oral absorptivity during oral administration or outstanding synergistic extension of the half-life period of concentration in blood. Furthermore, a study was conducted using a large number of HIV protease inhibitors known to possess high anti-virus activity to investigate suitable combinations of these compounds which exhibit synergistic increase in anti-virus activity and experiments were conducted based on this study. As a result, the present inventors have found that certain combinations of specific HIV protease inhibitors exhibit a remarkable synergistic increase in anti-virus activity. More specifically, studies have been conducted from this viewpoint relating to combinations of an HIV protease inhibitor which contains a compound abbreviated to KNI-272 (hereinafter defined) and another compound selected from a number of other HIV protease inhibitors. It was found that using a combination of the HIV protease inhibitor comprising KNI-272 and another specific HIV protease inhibitor can achieve a remarkable increase in synergistic medical treatment effect. This finding has led to the completion of the present invention.

Accordingly, an object of the present invention is to provide an AIDS therapeutic composition comprising, as essential effective components which bring about an HIV protease inhibition action, (R)-N-(1,1-dimethylethyl)-3-[(2S,3S)-2-hydroxy-3-[(R)-2-(5-isoquinolyloxyacetyl)amino-3-methylthiopropanoyl]amino-4-phenylbutanoyl]-1,3-thiazolidine-4-carboxyamide (KNI-272) and another compound selected from the following four HIV protease inhibitors: N-(1,1-dimethylethyl)-decahydro-2-[2(R)-hydroxy-4-phenyl-3(S)-{[N-(2-quinolylcarbonyl)-L-asparaginyl]amino}butyl]- (4aS, 8aS)-isoquinoline-3(S)-carboxyamide (Saquinavir), [5S-(5R*, 8R*, 10R*,11R*)]-10-hydroxy-2-methyl-5-(1-methylethyl)-1-[2-(1-methylethyl)-4-thiazolyl]-3,6-dioxo-8,11-bis(phenyl methyl)-2,4,7,12-tetraaza-tridecane-13-hydroxy acid 5-thiazolylmethyl ester (Ritonavir), [1(1S,2R),5(S)]-2,3,5-trideoxy-N-(2,3-dihydro-2-hydroxy-1H-indane-1-yl)-5-[2-(1,1-dimethylethyl) aminocarbonyl-4-(3-pyridinylmethyl)-1-piperazinyl]-2-(phenylmethyl)-D-erythropentonamide (Indinavir), and [3S-[2(2S*, 3S*), 3α, 4aβ, 8aβ ]]-N-(1,1-dimethylethyl)-decahydro-2-[2-hydroxy-3-[(3-hydroxy-2-methylbenzoyl)amino]-4-(phenylthio)butyl]-3-isoquinolinecarboxamide (Nelfinavir), in an amount and proportion in which a synergistic treatment effect can be exhibited. Although the treatment principle of the AIDS therapeutic agent of the present invention is based on the anti-virus activity originating from each of these HIV protease inhibitors used as indispensable active components, the treatment effect is synergistically provided by the combined use of these compounds.

### BRIEF DESCRIPTION OF THE DRAWINGS

Figure 1 shows the concentration range in which there is a synergistic increase in anti-HIV activity and the degree of increase achieved by the combined use of Saquinavir-methane sulfonate and KNI-272 as essential active components which bring about an HIV protease inhibitive action in Example 1.
Figure 2 shows the change of concentration in blood over time for KNI-272 when the KNI-272 was administered before or after oral administration of Ritonavir in Example 2.
Figure 3 shows the relationship between the concentrations of Ritonavir and KNI-272 in blood and anti-virus activity when these two compounds were used in combination in Example 3.
Figure 4 is a chart comparing changes in the concentrations of Indinavir and KNI-272 in plasma over time for the case in which these compounds were simultaneously administered orally to rats and the case in which these were individually administered in Example 4.
Figure 5 is a chart showing a comparison of changes in the concentration of KNI-272 in plasma over time for the case in which the KNI-272 was administered orally to rats one hour after administration of Indinavir and the case in which no Indinavir was administered in Example 4.
Figure 6 shows a chart comparing changes in the concentration of Nelfinavir in plasma over time for the case in which the Nelfinavir and KNI-272 were simultaneously administered orally to rats and the case in which only Nelfinavir was administered in Example 4.
Figure 7 shows a chart comparing changes in the concentration of KNI-272 in plasma over time for the case in which the Nelfinavir and KNI-272 were simultaneously administered orally to rats and the case in which only KNI-272 was administered in Example 4.

### BEST MODE OF THE PRESENT INVENTION

Two HIV protease inhibitors are administered in combination as essential components to bring about an HIV protease inhibitive action according to the AIDS therapeutic agent of the present invention. Specifically, there are four combinations, that is, KNI-272 and Saquinavir, KNI-272 and Ritonavir, KNI-272 and Indinavir, and KNI-272 and Nelfinavir. All these HIV protease inhibitors are known compounds developed as drugs to be orally administered. Accordingly, these compounds can be prepared by a synthetic method described in publications.

The compound generally called Saquinavir has a chemical structure shown by the following formula (I).

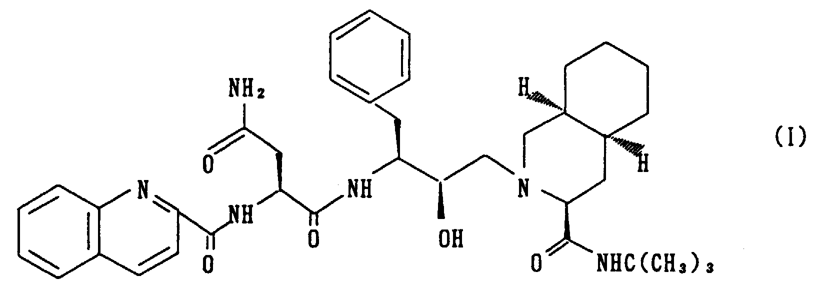

The nomenclature of the compound is N-(1,1-dimethylethyl)-decahydro-2-{2(R)-hydroxy-4-phenyl-3(S)-[[N-(2-quinolylcarbonyl)-L-asparaginyl]amino]butyl}-(4aS, 8aS)-isoquinoline-3(S)-carboxyamide. Saquinavir, which is a hydroxyethylamine-type derivative, is commercially available in the form of methanesulfonate (C₃₈H₅₀N₆O₅ · CH₄O₃S, molecular weight 766.96) as an HIV protease inhibitor for oral administration on the trademark INVIRASE (manufactured by Roche Co.). Saquinavir can be used in the present invention as a pharmaceutically acceptable salt, mainly as a salt of various acids, preferably as a methanesulfonate.

The compound generally called Ritonavir has a chemical structure shown by the following formula (II).

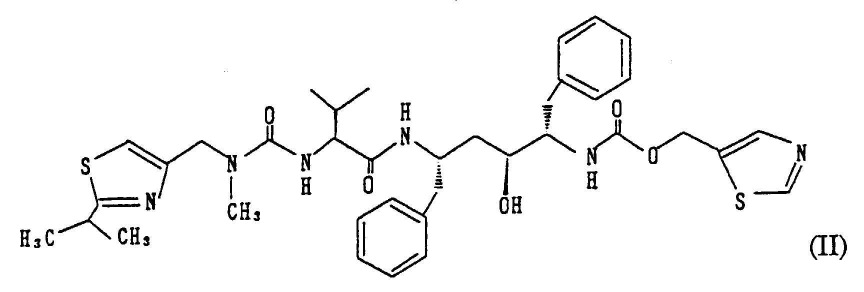

The nomenclature of the compound is [5S-(5R*, 8R*, 10R*,11R*)]-10-hydroxy-2-methyl-5-(1-methylethyl)-1-[2-(1-methylethyl)-4-thiazolyl]-3,6-dioxo-8,11-bis(phenyl methyl)-2,4,7,12-tetraaza-tridecane-13-hydroxyacid 5-thiazolylmethyl ester. This compound has a molecular formula of C₃₇H₄₈N₆O₅S₂ and a molecular weight of 720.95. This is grouped into one modification of a hydroxyethylamine-type derivative. Ritonavir is commercially available in the form of citrate as an HIV protease inhibitor for oral administration on the trademark NORVIR (manufactured by the Abbott Co.). Ritonavir can be used in the present invention as a pharmaceutically acceptable salt, mainly as a salt of various kinds of acid, preferably as a citrate.

The compound generally called Indinavir has a chemical structure shown by the following formula (III).

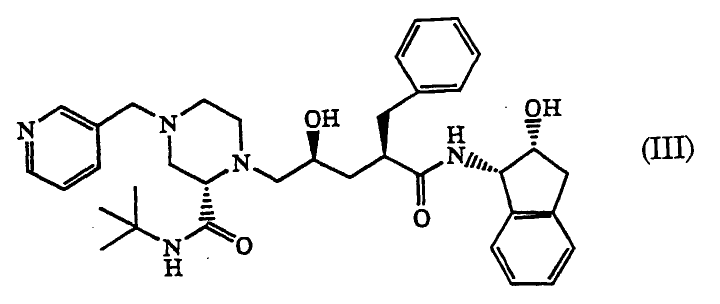

The nomenclature of the compound is [1(1S, 2R),5(S)]-2,3,5-trideoxy-N-(2,3-dihydro-2-hydroxy-1H-indane-1-yl)-5-[2-(1,1-dimethylethyl) aminocarbonyl-4-(3-pyridinylmethyl)-1-piperazinyl]-2-(phenylmethyl)-D-erythropentonamide. Indinavir is commercially available in the form of sulfate (C₃₆H₄₇N₅O₄ · H₂SO₄, molecular weight 711.88) as an HIV protease inhibitor for oral administration on the trademark CRIXIVAN (manufactured by Merck Co.). Indinavir can be used in the present invention as a pharmaceutically acceptable salt, mainly as a salt of various acids, preferably as a sulfate.

The compound generally called Nelfinavir has a chemical structure shown by the following formula (IV).

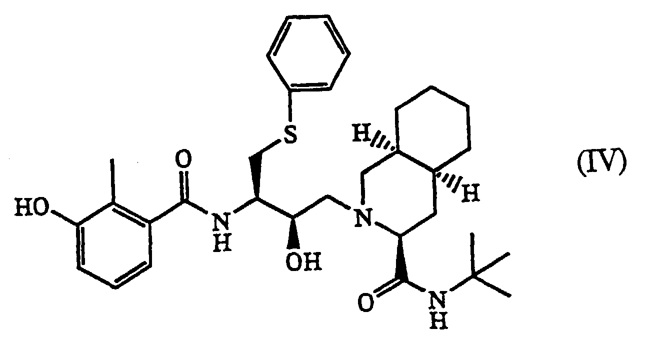

The nomenclature of the compound is [3S-[2(2S*, 3S*), 3α, 4aβ, 8aβ]]-N-1,1-dimethylethyl-decahydro-2-[2-hydroxy-3-[(3-hydroxy-2-methylbenzoyl)amino]-4-(phenylthio)butyl]-3-isoquinolinecarboxamide. Nelfinavir is commercially available in the form of methanesulfonate (molecular weight 663.90) as an HIV protease inhibitor for oral administration on the trademark VIRACEPT (manufactured by Agouron Co.). Nelfinavir can be used in the present invention as a pharmaceutically acceptable salt, mainly as a salt of various acids, preferably as a methanesulfonate.

The compound generally abbreviated to KNI-272 has a chemical structure shown by the following formula (V).

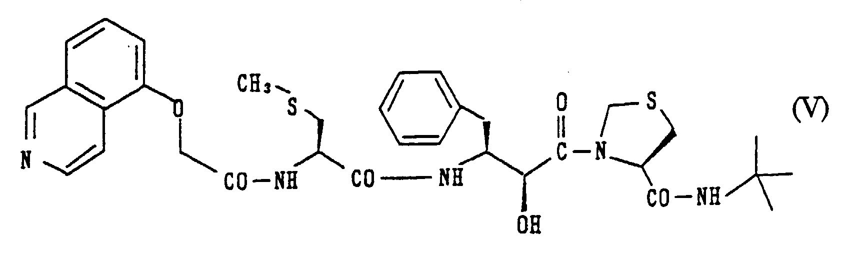

The nomenclature of the compound is (R)-N-1,1-dimethylethyl-3-[(2S,3S)-2-hydroxy-3-[(R)-2-(5-isoquinolyloxyacetyl)amino-3-methylthiopropanoyl]amino-4-phenylbutanoyl]-1,3-thiazolidine-4-carboxyamide. KNI-272 is a hydroxymethylcarboxamide-type derivative and can be used in the present invention as a pharmaceutically acceptable salt, mainly as a salt of various kinds of acid, such as a citrate.

In the AIDS therapeutic agent of the present invention, two HIV protease inhibitors are administered in combination as essential active components which bring about an HIV protease inhibitive action. These combinations are (1) KNI-272 and Saquinavir, (2) KNI-272 and Ritonavir, (3) KNI-272 and Indinavir, or (4) KNI-272 and Nelfinavir. Specifically, simultaneous administration of KNI-272 as the first compound and another drug as the second compound ensures that the therapeutic effects possessed by these two compounds are exhibited synergistically. The best modes for carrying out the present invention will now be described for each combination. In the following description, the anti-virus activity represents the results obtained by evaluating the activity of a wild virus strain which has not acquired resistance against these protease inhibitors, but not the results obtained using a variant virus strain which has acquired resistance against each of these compounds.

### (1) Combination of KNI-272 and Saquinavir

An embodiment of combined use of KNI-272 and Saquinavir as the AIDS therapeutic composition of the present invention will be discussed. In this combination of KNI-272 and Saquinavir, it is desirable that the amount of KNI-272 and Saquinavir used be less than the amount required for the individual active components to achieve 50% anti-virus activity with respect to the independent effect of each active component. Alternatively, it is desirable that the amount of KNI-272 and Saquinavir used be such that the sum of the anti-virus activity obtained by individual use of KNI-272 and that obtained by individual use of Saquinavir be less than 80%.

Specifically, it is desirable that the one time dosage comprises 3 to 25 mg/kg body weight of the subject (hereinafter the same) of Saquinavir and 3 to 15 mg/kg of KNI-272.

The medicaments, KNI-272 and Saquinavir, which are the essential active components in the AIDS therapeutic agent of the present invention, both exhibit the treatment effects by being dissolved in blood and caused to act on blood cells infected by HIV. Therefore, any administration route may be acceptable for these medicaments. Specifically, both the medicaments may be directly administered into a blood vessel or may be orally administered and caused to be absorbed into blood through the digestive organs. Alternatively, it is possible to orally administer one of the medicaments and to administer the other directly into a blood vessel. Irrespective of the method of administration, a method of administering KNI-272 and Saquinavir in amounts to provide concentrations in blood sufficient to enable these medicaments to synergistically exhibit the anti-virus activity which is a feature of the AIDS therapeutic agent of this embodiment should fall within the scope of the present invention.

For example, when KNI-272 and Saquinavir are orally administered, it is possible to prepare a composition in a dosage form to improve oral absorption, containing each of these compounds in a prescribed amount, then to combine two compounds in an amount and proportion appropriate for the pharmaceutical composition two compounds to exhibit a synergistic anti-virus activity. Because the time required for the compounds to be transferred into the blood after administration varies according to dosage forms, the compositions is not necessarily administered simultaneously. It is possible to set the administration time and intervals so that the concentrations in blood of both compounds are maintained in amounts and proportion ideal for these compounds to exhibit synergistic anti-virus activity.

The amount and proportion of KNI-272 and Saquinavir in which these compounds exhibit synergistic anti-virus activity should be the range wherein the anti-virus activity achieved by using both compounds is significantly higher than the sum of the anti-virus activity achieved by individual use of these compounds. Specifically, as shown by the experiment in Example 1 hereinafter, a preferable range of concentrations in blood of Saquinavir and KNI-272 which are used as essential active components to bring about the HIV protease inhibitive action in this embodiment should be selected from the range where the product of the concentrations of these two compounds, i.e [Saquinavir]x[KNI-272], is more than 100 (ng/ml)² when the concentrations of Saquinavir and KNI-272 are respectively limited to 1.25-20.0 ng/ml and 5.0-80.0 ng/ml; or more than 200 (ng/ml)² when the concentrations of Saquinavir and KNI-272 are respectively limited to 2.5-20.0 ng/ml and 10.0-80.0 ng/ml. If a dosage is selected from the range wherein the concentrations in blood of both compounds fall within the above-mentioned concentration range, a clearly synergistic anti-virus activity can be exhibited. An anti-virus activity higher than 50% can be achieved when the product of the concentrations [Saquinavir]x[KNI-272] is more than 100 (ng/ml)² and, particularly, an anti-virus activity higher than 80% can be achieved when the product of the concentrations [Saquinavir]x[KNI-272] is more than 200 (ng/ml)². Alternatively, the concentration ratio of the two compounds may be selected from the range [Saquinavir]:[KNI-272] = 1:64 to 2:1 when the concentrations of Saquinavir and KNI-272 are respectively limited to 1.25-20.0 ng/ml and 5.0-80.0 ng/ml; and more preferably from the range [Saquinavir]:[KNI-272] = 1:32 to 2:1 when the concentrations of Saquinavir and KNI-272 are respectively limited to 2.5-20.0 ng/ml and 10.0-80.0 ng/ml.

When the dosage of both compounds, Saquinavir and KNI-272, is greater than the amount required to individually produce a concentration in plasma sufficient to achieve an anti-virus activity exceeding 50%, there is no substantial synergistic pharmacology effect exhibited explicitly. Also, no substantial synergistic pharmacology effect is exhibited explicitly when the concentrations in plasma of these compounds are sufficient to achieve an anti-virus activity exceeding 95% as a simple arithmetical sum of the anti-virus activity of the two compounds.

It is necessary for the two compounds to explicitly exhibit a significant synergistic pharmacology effect to be used in an amount less than the amount in which the individual compound produces a concentration in plasma to achieve a 50% anti-virus activity individually or a 95% anti-virus activity as a simple arithmetical sum of the anti-virus activity of the two compounds.

If the concentration is selected from the range to produce this simple arithmetical sum of the anti-virus activity of the two compounds of 80% or less, the synergistic effect is outstanding.

The concentrations of Saquinavir and KNI-272 in blood decrease over time due to in vivo metabolism after administration. The case in which these compounds are administered in amounts to produce the above-mentioned blood concentration where no significant synergistic effect is exhibited immediately after administration is desirable because the concentration reaches the meaningful region due to in vivo metabolism with passage of time. In addition, if the total amount of the compounds required to achieve a desired anti-virus activity is significantly less than the amount of the individual compound when this compound is independently used, such a case is still medically valuable in view of a decrease in undesirable adverse effects, notwithstanding the fact that a synergistic increase which is attained exhibits no substantial significance. Specifically, the embodiment wherein Saquinavir and KNI-272 are administered in combination at a significantly reduced total dosage as compared with the case where Saquinavir or KNI-272 is individuality administered as an essential active component to exhibit an HIV protease inhibitive action at a higher dosage as commonly used, such as a case where production of infectious HIV virus particles is unusually accelerated for one or some reasons, belongs to the scope of the present invention.

### (2) Combination of KNI-272 and Ritonavir

An embodiment of combined use of KNI-272 and Ritonavir in the AIDS therapeutic agent of the present invention will be discussed. In this combination of KNI-272 and Ritonavir, it is desirable that the amount of KNI-272 and Ritonavir used be less than the amount required for the individual active components to achieve 50% anti-virus activity with respect to the independent effect of each active component. Alternatively, it is desirable in practice that the amount of KNI-272 and Ritonavir used be such that the sum of the anti-virus activity obtained by individual use of KNI-272 and that obtained by individual use of Ritonavir be less than 90%.

Specifically, it is desirable that the one time dosage comprises 3 to 15 mg/kg of Ritonavir and 3 to 15 mg/kg of KNI-272.

The medicaments, KNI-272 and Ritonavir, which are the essential active components in the AIDS therapeutic agent of the present invention both exhibit treatment effects by being dissolved in blood and caused to act on blood cells infected by HIV. Therefore, any administration route may be acceptable for these medicaments inasmuch as this effect is achieved. Specifically, both the medicaments may be directly administered into a blood vessel or may be orally administered and caused to be absorbed into blood through the digestive organs. Alternatively, it is possible to orally administer one of the medicaments and to administer the other directly into a blood vessel. Irrespective of the method of administration, a method of administerring KNI-272 and Ritonavir in amounts to provide concentrations in blood sufficient to enable these medicaments to synergistically exhibit the anti-virus activity which is a feature of the AIDS therapeutic agent of this embodiment should fall within the scope of the present invention. Specifically, the manner of administration by which a dosage and proportion realizing extension of the half-life of concentration in blood and an increase in oral absorptivity in oral administration are achieved by a synergistic effect belongs to the technical scope of the present invention. An increase in a synergistic treatment effect in the present embodiment means an increase in the treatment effect typified by relative increase in concentration in blood per unit dose due to extension of the half-life of concentration in blood or an increase in oral absorptivity which are synergistically caused by the simultaneous administration of the two compounds.

For example, when KNI-272 and Ritonavir are orally administered, it is possible to prepare a compositions with a dosage form to improve oral absorption, containing each of these compounds in a prescribed amount, then to combine two compounds in an amount and proportion appropriate for the pharmaceutical composition to exhibit a synergistic effect. Because the time required for the compounds to be transferred into the blood after administration varies according to the dosage form, the composition is not necessarily administered coincidentally. It is possible to set the administration time and interval so that the concentration in bloods of both compounds are maintained in amounts and proportion ideal for these compounds to exhibit synergistic extension of the half-life of concentration in blood and an increase in oral absorptivity.

The amount and proportion of KNI-272 and Ritonavir in which these compounds exhibit synergistic extension of the half-life of concentration in blood and an increase in oral absorptivity should be in the range wherein the effect achieved by using both compounds is significantly higher than the sum of the effects achieved by individual use of these compounds. Specifically, with regard to the MRT value which is an index of the half-life of concentration in blood, the value for KNI-272 (MRT=30 minutes) is markedly shorter than that for Ritonavir (MRT=180 minutes or more). The above amounts and proportions mean those required for the half-life of KNI-272 concentration in blood to be extended to a degree to be comparable with the half-life of Ritonavir, for instance, to as long as MET=90 minutes. The AUC value which is an index of the rate of oral absorptivity also increases in accordance with the extension of the half-life in concentration in blood of KNI-272. The above amounts and proportions also mean those required to synergistically increase not only the extension ratio of the half-life in concentration in blood, but also the AUC value for KNI-272 along with the increase in the maximum concentration in blood value (Cmax) of KNI-272. Because there is a significant difference between the half-life of Ritonavir concentration in blood and that of KNI-272, which are two compounds used as the essential active components exhibiting an HIV protease inhibitive action in the medicinal composition of the present invention, the synergistic effect in the extension of the half-life of concentration in blood or increase in the rate of oral absorptivity by the combined use of the two compounds is appears remarkably in KNI-272 for the most part. Although there is the same synergistic effect in Ritonavir, the effects of extension of the half-life in concentration in blood and increase in the rate of oral absorptivity appear relatively very small, because the half-life in concentration in blood of Ritonavir is inherently very long.

In addition, as shown in the experiment in Example 3 hereinafter, when the anti-virus activity in vitro was measured for the two compounds, Ritonavir and KNI-272, which are used as essential active components to exhibit an HIV protease inhibitive action in the AIDS therapeutic agent of this embodiment, it has been proven that the combined use of both compounds shows a synergistic increase in the anti-virus activity. Specifically, when a simple arithmetic addition of the values of anti-virus activity achieved in a case where each compound was individually used and the value for anti-virus activity achieved in a case where these two compounds were used together in the same concentrations are compared, the latter value was proven to be superior.

Although this increase in the synergistic anti-virus activity in vitro does not affect the above-mentioned extension of the half-life of concentration in blood or increase in the rate of oral absorptivity, the increase in the treatment effect in vivo is a product of the synergistic effects brought about by the combined use of the two compounds, indicating that the increase in the synergistic anti-virus activity, ultimately results in a remarkable improvement. Thus, the anti-virus activity individually exhibited by Ritonavir or KNI-272 either synergistically increases by the combined use of the two compounds or at least does not impair the anti-virus activity of the other. Therefore, when the AIDS therapeutic agent of this embodiment is used, a treatment effect which exceeds a simple addition of the treatment effects (anti-virus activity) in concentration in blood of each compound accompanied by a synergistic extension of the half-life of concentration in blood which is achieved by the administration of both compounds, or an increase in the rate of oral absorptivity is obtained.

In addition, it is needless to mention that no synergistic pharmacological effects can be substantially obtained if the individual amount of Ritonavir and KNI-272 administered is selected from a range sufficient to cause the concentration in blood of each chemical to exhibit 50% or more anti-virus activity. It is also needless to mention that no synergistic pharmacological effects can be substantial expected if the total amount of Ritonavir and KNI-272 administered is selected from a range sufficient to cause the concentration in bloods of the chemicals to exhibit 95% or more anti-virus activity as a simple arithmetical sum of the anti-virus activity of the two chemicals. It is necessary for the two compounds to explicitly exhibit a significant synergistic pharmacology effect to be used in an amount less than the amount in which the individual compound produces a concentration in plasma to achieve a 50% anti-virus activity individually or a 95% anti-virus activity as a simple arithmetical sum of the anti-virus activity of the two compounds. If the concentration is selected from a range which makes this simple arithmetical sum of the anti-virus activity of the two compounds 90% or less, the synergistic effect is outstanding.

Specifically, a preferable range of concentration in bloods should be selected from the range where the product of the concentration in bloods of these two compounds, i.e [Ritonavir]x[KNI-272], is more than 800 (ng/ml)², more preferably more than 1200 (ng/ml)², when the concentrations of Ritonavir and KNI-272 are respectively limited to 20.0-40.0 ng/ml and 20.0-80.0 ng/ml. Alternatively, the concentration ratio of the two compounds should be selected from the range [Ritonavir]:[KNI-272] = 1:4 to 2:1 when the concentrations of Ritonavir and KNI-272 are respectively limited to 20.0-40.0 ng/ml and 10.0-80.0 ng/ml; more preferably from the range [Ritonavir]:[KNI-272] = 1:2 to 2:1 when the concentrations of Ritonavir and KNI-272 are respectively limited to 20.0-40.0 ng/ml and 20.0-80.0 ng/ml.

Because the concentration in bloods of both compounds, Ritonavir and KNI-272, change over time due to in vivo metabolism or excretion, the above-mentioned dose of individual compound to be administered is determined based on the amount in which each chemical, if independently administered, reaches a desired concentration after a prescribed period of time. This prescribed period of time is determined individually for each compound. In this embodiment, this period is corresponding to the value of MRT which is the index for the half-life of concentration in blood when each chemical is intravenously administered individually.

Although the concentration in bloods of these compounds, Ritonavir and KNI-272, decrease over time after administration due to in vivo metabolism, the concentration immediately after administration is in the range where no significant synergistic anti-virus activity can be exhibited due to an increase in the maximum concentration value in blood (Cmax) which is brought about by the combined use. As the time is past, the concentration reaches a region where there is great significance in respect of the synergistic effect of anti-virus activity due to in vivo metabolism. However, because of the synergistic extension of the half-life of concentration in blood the time required for the concentration of compounds to reach this region, as well as the period of time required for the concentration to become less than this region is markedly prolonged. The concentration immediately after administration where no significant synergistic anti-virus activity can be exhibited still remains a preferred embodiment of the present invention inasmuch as an treatment effect can be achieved. In addition, if the total amount of the compounds required to achieve a desired anti-virus activity is significantly less than the amount of the individual compound when this compound is independently used, such a case is still medically valuable in view of a decrease in undesirable adverse effects, notwithstanding the fact that the synergistic increase which is attained exhibits no substantial significance. Specifically, the embodiment wherein Ritonavir and KNI-272 are administered in combination at a significantly reduced total dosage as compared with the case where Ritonavir or KNI-272 is individually administered as an essential active component to exhibit an HIV protease inhibitive action at a higher dosage as commonly used, such as a case where production of infectious HIV virus particles is unusually accelerated for one or some reasons, is within the scope of the present invention.

### (3) Combination of KNI-272 and Indinavir

An embodiment of combined use of KNI-272 and Indinavir in the AIDS therapeutic agent of the present invention will now be discussed. In this combination of KNI-272 and Indinavir, it is desirable that the amount of KNI-272 and Indinavir used be less than the amount required for individual active components to achieve 50% anti-virus activity with respect to the independent effect of each active component. Alternatively, it is desirable that the amount of KNI-272 and Indinavir used be such that the sum of the anti-virus activity obtained by individual use of KNI-272 and that obtained by individual use of Indinavir be less than 90%. Specifically, it is desirable that the one time dosage comprises 3 to 25 mg/kg, in particular 5 to 15 mg/kg, of Indinavir and 3 to 20 mg/kg, in particular 3 to 15 mg/kg, of KNI-272.

The medicaments, KNI-272 and Indinavir, which are the essential active components in the AIDS therapeutic agent of the present invention both exhibit treatment effects by being dissolved in blood and caused to act on blood cells infected by HIV. Therefore, any administration route may be acceptable for these medicaments inasmuch as this effect is achieved. A preferred method of administration is to give both medicaments orally and cause them to be absorbed into the blood through the digestive organs. Alternatively, it is possible to administer one of the medicaments directly into a blood vessel and the other orally. Specifically, it is possible to administer Indinavir into a blood vessel, then orally administer KNI-272; or to administer KNI-272 into a blood vessel, then orally administer Indinavir. Irrespective of the method of administration, a method of providing KNI-272 and Indinavir in amounts to cause the concentration in bloods to enable these medicaments to synergistically exhibit the anti-virus activity which is a feature of the AIDS therapeutic agent of this embodiment should fall within the scope of the present invention.

For example, when KNI-272 and Indinavir are orally administered, it is possible to prepare a composition with a dosage form to improve oral absorption, containing each of these compounds in a prescribed amount, then to combine two compounds in an amount and proportion appropriate for the pharmaceutical composition to exhibit a synergistic anti-virus activity. Because the time required for the compounds to be transferred into the blood after administration varies according to the dosage form, the composition is not necessarily administered coincidentally. It is possible to set the administration time and interval so that the concentration in bloods of both compounds are maintained in amounts and a proportion ideal for these compounds to exhibit synergistic anti-virus activity. Specifically, KNI-272 and Indinavir may be orally administered simultaneously, but preferably Indinavir is first administered orally, then after the period of time required for Indinavir to be transferred into the blood, i.e. about 30 minutes to two hours later, KNI-272 is orally administered.

The amount and proportion of KNI-272 and Indinavir in which these compounds exhibit synergistic anti-virus activity should be in a range wherein the anti-virus activity achieved by using both compounds is significantly higher than the sum of the anti-virus activity achieved by individual use of these compounds. Specifically, as shown by the experiment in Example 5 hereinafter, a preferable range of concentration in bloods of Indinavir and KNI-272 which are used as essential active components to bring about the HIV protease inhibitive action in this embodiment should be selected from a range wherein the product of concentrations of these two compounds, i.e [Indinavir]x[KNI-272], is more than 1600 (ng/ml)², and more preferably more than 3200 (ng/ml)², when the concentrations of Indinavir and KNI-272 are respectively limited to 1.25-50.0 ng/ml and 20.0-80.0 ng/ml. If a dosage is selected from a range wherein the concentration in bloods of both compounds fall within the above-mentioned concentration range, a clearly synergistic anti-virus activity can be exhibited. An anti-virus activity higher than 50% can be achieved when the product of the concentrations [Indinavir]x[KNI-272] is more than 1600 (ng/ml)² and, particularly, an anti-virus activity higher than 80% can be achieved when the product of the concentrations [Indinavir]x[KNI-272] is more than 3200 (ng/ml)². In addition to these conditions, it is desirable that the concentration ratio of the two compounds be selected from the range [Indinavir]:[KNI-272] = 1:4 to 2:1, particularly from the range [Indinavir]:[KNI-272] = 1:2 to 2:1.

When the dosage of both compounds, Indinavir and KNI-272, is greater than the amount required to individually produce a concentration in blood sufficient to achieve an anti-virus activity exceeding 50%, or the concentrations of these compounds are sufficient to achieve an anti-virus activity exceeding 95% of a simple arithmetical sum of the anti-virus activity of the two compounds, there is no substantial synergistic pharmacology effect exhibited explicitly. However, in common the concentrations of Indinavir and KNI-272 in blood decrease over time after administration due to in vivo metabolism and excretion. Therefore, the concentrations of the medicaments in blood can be maintained in a range in which a treatment effect is exhibited by administering the medicine in portions at almost equal intervals, usually, three or four times a day. The concentration range in which the above-mentioned medicaments exhibit a synergistic effect should be the point where the concentration in blood of each medicament is the lowermost, that is, the concentration in blood immediately before the next administration should fall within the above-mentioned concentration region. On the other hand, it is needless to mention that although the concentration in blood of each medicament shows a maximum value immediately after administration and then decreases thereafter, no synergistic pharmacology effects can be exhibited during a considerable period of time between the administration intervals. In addition, if the total amount of compounds required to achieve a desired anti-virus activity is significantly less than the amount of the individual compound when this compound is independently used, such a case is still medically valuable in view of a decrease in undesirable adverse effects, notwithstanding the fact that the synergistic increase achieved exhibits no substantial significance.

On the other hand, in the combined use of Indinavir and KNI-272 in the AIDS therapeutic agent of this embodiment the synergistic treatment effect can be obtained not only from the above-mentioned synergistic increase in the anti-virus activity, but also from noticeable improvement in bio-availability during oral administration, such as a remarkable increase in digestive tract absorption and an eminent increase in the maximum concentration value in blood (Cmax) caused by retardation in concentration in blood attenuation as shown later in Example 4. In other words, the concentration in blood achieved by combined use of the medicaments is higher than the same amounts are administered individually due to the acute increase the maximum concentration value in blood (Cmax), and this brings about a synergistic increase in anti-virus activity, as well as a further synergistic increase in the treatment effect.

To achieve this eminent increase in the maximum concentration value in blood maximum value (Cmax), Indinavir and the compound of KNI-272 are orally administered simultaneously or Indinavir is orally administered first and then the compound of KNI-272 is orally administered within the time when the concentration in blood of Indinavir reaches maximum. Either method of administration is acceptable. When Indinavir is orally administered first, the compound of KNI-272 should be orally administered between the time when absorption of Indinavir becomes manifest, which usually takes place within 30 minutes after administration, and the time when the concentration in blood of Indinavir reaches maximum, which may be two hours after administration. Usually the time difference between the administration of the two medicaments is about one hour. Taking the case where Indinavir and the compound of KNI-272 are administered to a human at intervals of 8 hours, the dose ratio (by weight) of Indinavir and KNI-272 is preferably selected from the range Indinavir:KNI-272 = 1:4 to 9:1, and more preferably 1:2 to 4:1.

### (4) Combination of KNI-272 and Nelfinavir

An embodiment of combined use of KNI-272 and Nelfinavir as the AIDS therapeutic agent of the present invention will be discussed. Also in this embodiment, when both KNI-272 and Nelfinavir are orally administered, a desirable one time dosage comprises 3 to 20 mg/kg, particularly 3 to 15 mg/kg, of Nelfinavir and 3 to 20 mg/kg, particularly 3 to 15 mg/kg, of KNI-272. This dosage of KNI-272 and Nelfinavir may be orally administered simultaneously, but preferably Nelfinavir is first administered orally, then after the time required for Nelfinavir to be transferred into the blood, i.e. about 30 minutes to three hours later, KNI-272 is orally administered.

The amount and proportion of KNI-272 and Nelfinavir in which these compounds exhibit synergistic anti-virus activity should in usual be in the range wherein the anti-virus activity achieved by using both compounds is significantly higher than the sum of the anti-virus activity achieved by individual use of these compounds. Specifically, with respect to the concentration ratio of the two compounds, such a ratio may be selected from the range [Nelfinavir]:[KNI-272] = 1:4 to 2:1, and more preferably from the range [Nelfinavir]:[KNI-272] = 1:2 to 2:1. When the dosage of both compounds, Nelfinavir and KNI-272, is greater than the amount to individually produce a concentration in blood sufficient to achieve an anti-virus activity exceeding 50%, or the concentrations of these compounds are sufficient to achieve an anti-virus activity exceeding 95% as a simple arithmetical sum of the anti-virus activity of the two compounds, there is no substantial synergistic pharmacology effect exhibited explicitly. However, the concentrations of Nelfinavir and KNI-272 in blood decrease over time after administration due to in vivo metabolism and excretion. Therefore, the concentrations of the medicaments in the blood can be maintained in the range in which the treatment effect is exhibited by administering the medicine in portions at almost equal intervals, usually, three or four times a day. The concentration range in which the above-mentioned medicaments exhibit a synergistic effect should be the point where the concentration in blood of each medicament is the lowermost, that is, the concentration in blood immediately before the next administration should fall within the above-mentioned concentration region.

In the combined use of Nelfinavir and KNI-272 in the AIDS therapeutic agent of this embodiment the synergistic treatment effect can be obtained not only from the synergistic increase in the anti-virus activity, but also from improvement in bio-availability during oral administration, such as an increase in digestive tract absorption and a substantial increase in the maximum concentration in blood value (Cmax) caused by retardation in concentration in blood attenuation as shown later in Example 4. In other words, the concentration in blood achieved by combined use of the medicaments is higher than the case where the same amounts are administered individually due to the increase in the maximum concentration value in blood (Cmax). This brings about a further synergistic increase anti-virus activity, as well as a synergistic increase in the treatment effect.

To achieve this eminent increase in the maximum concentration value in blood (Cmax), Nelfinavir and the compound of KNI-272 are orally administered simultaneously or Nelfinavir is orally administered first and then the compound of KNI-272 is orally administered within the time when the concentration in blood of Nelfinavir reaches a peak. Either method of administration is acceptable. When Nelfinavir is orally administered first, the compound of KNI-272 should be orally administered between the time when absorption of Nelfinavir becomes manifest, which usually takes place within 30 minutes after administration, and the time when the concentration in blood of Nelfinavir reaches a maximum, which may be three hours after administration. Usually the time difference between the administration of the two medicaments is about one to two hours. Taking the case where Nelfinavir and the compound of KNI-272 are administered to a human being at intervals of 8 hours, the dose ratio (by weight) of Nelfinavir and KNI-272 is preferably selected from the range Nelfinavir:KNI-272 = 1:4 to 8:1, and more preferably 1:2 to 4:1.

In the AIDS therapeutic agent of the present invention, in addition to the combinations of two compounds which are used as the essential active components exhibiting an HIV protease inhibitive action, i.e. the combinations of KNI-272 and Saquinavir, KNI-272 and Ritonavir, KNI-272 and Indinavir, and KNI-272 and Nelfinavir, a nucleic acid derivative-type reverse transcription enzyme inhibitor, such as AZT (azidothymidine), dideoxycytidine (DDC), dideoxyinosine (DDI), or the like, may be used. That is, in addition to the synergistic increase in the anti-virus activity originating from the HIV protease inhibitive action in the present invention, it is possible to have an additional increase in the anti-virus activity by the combined use of a third medicament, in which different mechanisms, one an HIV protease inhibitor and the other a conventionally known reverse transcription enzyme inhibitor, are utilized. In this instance, because the mechanism for producing the anti-virus activity differs from the mechanism for producing the HIV protease inhibitive action, the synergistic increase in the anti-virus activity itself, which is a feature of the present invention, of course is not affected by the presence of the reverse transcription enzyme inhibitor.

The AIDS therapeutic agent of the present invention comprising two essential active components which bring about an HIV protease inhibitive action, that is, a combination of KNI-272 and Saquinavir, KNI-272 and Ritonavir, KNI-272 and Indinavir, or KNI-272 and Nelfinavir, can be administered at the above-described suitable dosage and proportion to treat patients already suffered from AIDS. When this agent is used as a ternary medicinal composition by the addition of a reverse transcription enzyme inhibitor, the agent can be used of a less dosage that satisfies the above-mentioned proportion. Particularly, when the agent is administered to prevent AIDS symptoms from manifesting in HIV infected patients, the dosage may be less, inasmuch as the above-mentioned proportion of each medicament is satisfied.

Although the dosage and method of administration of this type of medicine utilizing an anti-virus activity by inhibiting formation of virus particles in HIV infected cells are appropriately selected according to the purpose of administration, symptoms of the patient, age, and so on, it is desirable in the present invention that at least KNI-272 be orally administered. In addition, because the period of administration may be long and continuous when this agent is used with the objective of preventing AIDS symptoms from manifesting, treating AIDS patients suffering from manifesting symptoms, or retarding progress of the disease, both medicaments are preferably administered orally for ease of administration to the patients.

In addition, taking into consideration the fact that one of the combination among the combinations of KNI-272 and Saquinavir, KNI-272 and Ritonavir, KNI-272 and Indinavir, and KNI-272 and Nelfinavir, as well as at the above-described suitable dosage and proportion, is selected according to the objective of administration, it is preferable to prepare compositions each containing an individual compound and then to prepare a medicinal composition from these compositions, suitably formulating each composition according to an appropriate dosage and proportion.

Furthermore, each individual active component has a different in vivo metabolism, specifically, the half-lifes of concentration in blood of Ritonavir, Indinavir, and Nelfinavir are longer than the half-life of concentration in blood of KNI-272, and the extension of the half-life of concentration in blood of KNI-272 and increase in the maximum value of KNI-272 are brought about by the synergistic effect of parallel use of Ritonavir, Indinavir, or Nelfinavir. Taking this into consideration, when a dosage of any one of Ritonavir, Indinavir, or Nelfinavir is higher than the dosage of KNI-272, a method of administration by which Ritonavir, Indinavir, or Nelfinavir is first transferred into the blood and then KNI-272 is orally administered is desirable. Conversely, when the dosage of Ritonavir, Indinavir, or Nelfinavir is less than the dosage of KNI-272, the method of orally administering both components at the same time is desirable.

The present invention will be explained in more detail by way of examples, which are not intended to be limiting of the present invention.

### Example 1

### The first embodiment of the AIDS therapeutic agent of the present invention

An increase in the synergistic anti-virus activity in the combined use of KNI-272 and Saquinavir is shown by a specific experimental example. The anti-virus activity was evaluated as the capability of controlling generation of infectious virus particles in vitro. The effect of cell extinction to HIV infection was calculated when Saquinavir methanesulfonate and KNI-272 respectively or both were added to a culture liquid, to determine the concentration region where a synergistic increase in the anti-virus activity in the combined use of the two compounds is conspicuous. Measurement of the anti-virus activity in vitro was carried out according to the following procedure. In the description hereinafter the concentration of methanesulfonate is indicated as the concentration converted into Saquinavir. The concentration of KNI-272 is indicated as the concentration of the compound itself.

### Measurement of anti-HIV activity in vitro

A IIIB strain of HIV-1 was used as an HIV wild strain and CEM-SS cells were used as infected cells in the above-mentioned culture. The amount of HIV sufficient to kill all CEM-SS cells 6 days after inoculation (multiplicy of infection, MOI of 0.01-0.05) was determined and this amount of HIV was added. A 96 well micro-titre plate was used to inoculate 5x10³ CEM-SS cells per well in this case. At the time of inoculation of cells, a test compound was added to the culture medium to a prescribed concentration. Three specimens were used for each test level. The following controls were used (1) as colorimetric controls: a sample with only a test compound added to the culture medium, (2) as a cytotoxic control for a test compound: a sample with only cells and the test compound added to the culture medium, (3) as a virus control: a sample with the cells inoculated with only a virus, and (4) as a cell survival control, a sample consisting only of cells. In addition, AZT and DDC which are compounds with a known anti-HIV activity were used as positive controls. After the cells were added, they were inoculated with the infecting virus, followed by incubation at 37°C for 6 days. Cellular denaturalization due to HIV infection was calorimetrically measured for each well. Employing the colorimetry, was the metabolism from XTT Tetrazolium to XTT Formazan was evaluated. This colorimetry is called the XTT method (see Weislow et al., Journal of the National Cancer Institute 81, 577-586 (1989)). After the above-mentioned incubation, a prescribed amount of XTT was added to each well and incubation was continued for a further four hours at 37°C to measure the amount of XTT Formazan produced by metabolism by means of calorimetric quantitation.

The anti-HIV activity for each experimental level was calculated by comparing the number of undenatured cells measured by the above-mentioned assay with the corresponding number of cells in the four control groups. Specifically, the proportion of cellular denaturalization due to HIV infection controlled by the addition of the test compound was calculated. A systematic deviation between the several experiments was corrected based on the results of AZT and ddC used as positive controls.

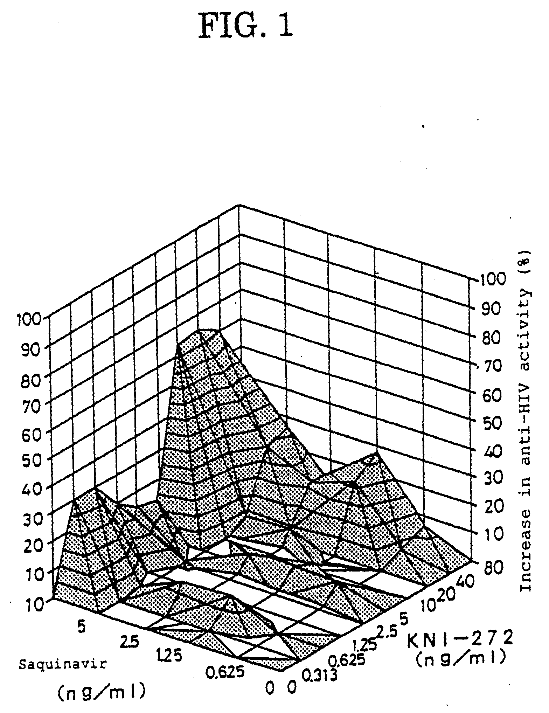

In this example, 0 ng/ml and several concentrations in the range of 0.625-10.0 ng/ml of Saquinavir methanesulfonate and 0 ng/ml and several concentrations in the range of 0.313-80.0 ng/ml of KNI-272 were used. The results (anti-HIV activities) obtained under these conditions using either one of these medicaments or both medicaments in combination are shown in Table 1. Table 2 shows the results calculated by subtracting the sum of anti-HIV activity values at given concentrations of the two medicaments front the anti-HIV activity value obtained by the combined use of the two medicaments at the same concentration. The concentration region in which this difference shows a positive value is judged to exhibit a synergistic increase in the anti-HIV activity. The results of Table 2 are also diagrammatically shown in Figure 1.

**TABLE 1**

| KNI-272 (ng/ml) | Anti-HIV activity (%) | | | | | |
|---|---|---|---|---|---|---|
| | Saquinavir methanesulfonate (ng/ml) | | | | | |
| | 0 | 0.625 | 1.25 | 2.5 | 5.0 | 10.0 |
| 0 | 0 | 3.4 | 0.0 | 2.8 | 7.9 | 13.0 |
| 0.313 | 4.8 | 5.1 | 5.2 | 7.3 | 12.4 | 46.5 |
| 0.625 | 0.0 | 3.7 | 8.1 | 9.9 | 8.5 | 43.8 |
| 1.25 | 1.2 | 8.7 | 6.4 | 10.2 | 10.9 | 44.9 |
| 2.5 | 11.0 | 12.0 | 9.1 | 10.7 | 20.0 | 38.4 |
| 5.0 | 8.5 | 16.9 | 13.6 | 15.5 | 10.1 | 33.6 |
| 10.0 | 13.1 | 7.9 | 14.5 | 10.0 | 20.1 | 89.4 |
| 20.0 | 15.7 | 18.8 | 13.8 | 22.5 | 26.6 | 93.3 |
| 40.0 | 27.6 | 33.5 | 49.3 | 46.7 | 59.7 | 99.9 |
| 80.0 | 65.7 | 75.8 | 93.3 | 80.2 | 100 | 100 |

**TABLE 2**

| KNI-272 (ng/ml) | Increase in anti-HIV activity (%) due to combined use | | | | |
|---|---|---|---|---|---|
| | Saquinavir methanesulfonate (ng/ml) | | | | |
| | 0.625 | 1.25 | 2.5 | 5.0 | 10.0 |
| 0.313 | -3.1 | 0.4 | -0.3 | -0.3 | 28.7 |
| 0.625 | 0.3 | 8.1 | 7.1 | 0.6 | 30.8 |
| 1.25 | -6.7 | -5.6 | -4.6 | -9.0 | 19.9 |
| 2.5 | -2.4 | -1.9 | -3.1 | 1.1 | 14.4 |
| 5.0 | 5.0 | 5.1 | 4.2 | -6.3 | 12.1 |
| 10.0 | -8.6 | 1.4 | -5.9 | -0.9 | 63.3 |
| 20.0 | -0.3 | -1.9 | 4.0 | 3.0 | 64.6 |
| 40.0 | 2.5 | 21.7 | 16.3 | 24.2 | 59.9 |
| 80.0 | 6.7 | 27.6 | 11.7 | 26.4 | 21.3 |

Under the above-mentioned experimental conditions used in this example, it was seemingly found that there was no synergistic increase in the range wherein a simple arithmetic sum of the control rate achieved by independent addition of these compounds exceeds 95%. With regard to this concentration region wherein no synergistic increase can be seemingly identified, a similar evaluation was carried out adding a larger amount of HIV to qualitatively evaluate the presence or absence of a synergistic effect. Because the amount of HIV inoculated is too high for the virus control to exhibit a true value, non-infected cells (cells which are not denaturalized) in the virus control are exhausted within a much shorter period than six days. This affords qualitative results rather than quantitative results.

An can be understood from the results shown in Table 1 and Table 2, when Saquinavir methanesulfonate was added in the amount of 10.0 ng/ml, a synergistic increase was seen with the addition of KNI-272 in an amount of 0.313-80.0 ng/ml. When the amount of Saquinavir methanesulfonate was 1.25-5.0 ng/ml, a clear synergistic increase was seen when 40.0-80.0 ng/ml of KNI-272 was added. The results of the synergistic increase obtained by the addition of 80.0 ng/ml of KNI-272 appeared. However, another experiments using 80.0 ng/ml of KNI-272, but adding a larger amount of HIV, confirmed a synergistic effect comparable to the case in which 40.0 ng/ml of KNI-272 was used, although the results were qualitative. Similarly, when Saquinavir methanesulfonate was added in the amount of 20.0 ng/ml, a synergistic increase was confirmed qualitatively when 0.313-80.0 ng/ml of KNI-272 was used.

These results clearly confirm a synergistic increase in the anti-HIV activity by combined use of Saquinavir methanesulfonate and KNI-272. This is more clearly understood from the total sum of the volume of the part shaded by oblique lines in Figure 1. That is, the calculated value of 200 µM²% or larger in this chart is much more than 100 µM²% which is considered to be an index representing a remarkable synergistic increase in anti-HIV activity (Antimicrobial Agents and Chemotheraphy, 39, 2718-2727 (1995) and so on reference), proving a manifest synergistic increase resulting from the combined use of the two medicaments. Comparing these results with the case where these medicaments are individually used, an anti-HIV activity equivalent to that obtained when only 10.0 ng/ml of Saquinavir methanesulfonate is added can be obtained by using about 10.0-20.0 ng/ml of KNI-272. It is surprising that addition of KNI-272 in an amount of 30.0 ng/ml, which is equivalent to the total sum of 10.0+20.0 ng/ml, exhibits only 1/3 of the anti-HIV activity that exhibited by the combined use of 10.0 ng/ml of Saquinavir methanesulfonate and 20.0 ng/ml of KNI-272.

Although it is true that the combined use of Saquinavir methanesulfonate and KNI-272 brings about a synergistic increase in anti-HIV activity in a broad concentration range, in order for a manifest AIDS treatment effect to be achieved by using only essential active components exhibiting HIV protease inhibitive action, a practical concentration range should be selected from the range in which about 50% or more anti-HIV activity can be achieved. Taking this factor into consideration, useful concentration ranges are as follows. Specifically, when Saquinavir methanesulfonate is added in an amount from 10.0-20.0 ng/ml, the amount of KNI-272 is selected from the range of 10.0-80.0 ng/ml; when Saquinavir methanesulfonate is added in an amount of 2.5-10.0 ng/ml, the amount of KNI-272 is selected from the range of 40.0-80.0 ng/ml; and when Saquinavir methanesulfonate is added in an amount of 1.25-2.5 ng/ml, the amount of KNI-272 is 80.0 ng/ml. More preferably, if a synergistic effect realizing an anti-HIV activity exceeding about 80% is desired, the concentration region to be selected should be as follows. That is, when Saquinavir methanesulfonate is added in an amount of 10.0-20.0 ng/ml, the amount of KNI-272 is selected from the range of 10.0-80.0 ng/ml; when Saquinavir methanesulfonate is added in an amount of 1.25-10.0 ng/ml, the amount of KNI-272 is 80.0 ng/ml.

The above concentration range can be generalized by the product of the concentrations of Saquinavir methanesulfonate and KNI-272. Specifically, when the amounts of these components to be added are limited to the ranges of 1.25-20.0 ng/ml and 5.0-80.0 ng/ml, respectively, the product, [Saquinavir methanesulfonate]x[KNI-272] should be more than 100 (ng/ml)²; and when the amounts of Saquinavir methanesulfonate and KNI-272 are limited to the ranges of 2.5-20.0 ng/ml and 10.0-80.0 ng/ml, respectively, [Saquinavir methanesulfonate]x[KNI-272] should be selected from the range exceeding 200 (ng/ml)². Alternatively, defining the added amount in terms of the concentration ratio of the two compounds, when the amount of Saquinavir to be added is limited to the range of 1.25-20.0 ng/ml and that of KNI-272 to 5.0-80.0ng/ml, the concentration ratio [Saquinavir]:[KNI-272] should be 1:64 to 2:1; and particularly when the amounts of Saquinavir and KNI-272 to be added are limited to the range of 2.5-20.0 ng/ml and 10.0-80.0 ng/ml, respectively, the concentration ratio [Saquinavir]:[KNI-272] should be in the range of 1:32-2:1.

It is unnecessary to mention that when a lower anti-HIV activity is acceptable, such as the case where the purpose of administration is to prevent AIDS symptoms from being manifested, a smaller value of the product [Saquinavir methanesulfonate]x[KNI-272] can be applied. A lower anti-virus activity induced by HIV protease inhibitive action can be used also in the a three-component drug composition in which a reverse transcription enzyme inhibitor is incorporated in addition to the essential active components exhibiting the HIV protease inhibitive action. Specifically, when the amounts of Saquinavir and KNI-272 are limited to the ranges of 1.25-20.0 ng/ml and 5.0-80.0 ng/ml, respectively, the product [Saquinavir methanesulfonate]x[KNI-272] larger than 100 (ng/ml)² is an ideal amount for the three-component composition containing a reverse transcription enzyme inhibitor in addition to the essential active components exhibiting the HIV protease inhibitive action in view of the fact that an anti-virus activity exceeding 50% as well as a synergistic increase of the effects are exhibited.

### Example 2

A synergistic extension of the half-life of concentration in blood and increase in the rate of oral absorptivity achieved by the second embodiment, which is parallel administration of KNI-272 and Ritonavir, in the AIDS therapeutic agent of the present invention will be described by way of a specific experimental example. In this experiment, the half-life of concentration in blood and the rate of oral absorptivity of KNI-272 and Ritonavir, when these medicaments are individually administered, were first compared. It was confirmed that with regard to the MRT value which is an index of the half-life of concentration in blood, KNI-272 has a value (MRT: 30 minutes) much lower than Ritonavir (MRT: more than 180 minutes). Taking this into consideration, major attention was paid to the change in concentration in blood of KNI-272 over time to evaluate presence or absence of a synergistic extension of the half-life of concentration in blood or a synergistic increase in the rate of oral absorptivity by the combined use of these compounds.

The following drug interactions are generally given as main factors causing fluctuations of dynamics in vivo:
(1) interaction relating to drug absorption,
(2) interaction relating to plasma protein binding,
(3) interaction induced by a transporting carrier when passing through a biological membrane (such as intake into liver or kidney, bile, excretion to urine), and
(4) interaction occurring during a metabolism.

Among these, the interaction (1) causes reduction of the rate of oral absorptivity during oral administration and the interaction (2) has almost no effect on the concentration in plasma, typically the concentration of non-binding type medicines. The interactions (3) and (4) are not affected by the manner of administration, but exhibit some effect on the drug interaction in deviation of the dynamics in vivo.

To evaluate a extension of the half-life of concentration in blood and increase in the rate of oral absorptivity while excluding systematic deviation factors such as deviation of fluctuation of dynamics in vivo during combined use, the effects of interactions (1) and (2) can be eliminated by administering the two medicines at a certain interval.

In this example, the change in concentration in blood of KNI-272 over time when the KNI-272 was orally administered in combination with Ritonavir, i.e. the change in the concentration of a non-binding type drug in plasma over time, was measured to evaluate the effect of drug interactions on fluctuation of dynamics in vivo. Capsules for oral administration, containing 100 mg of Ritonavir each, which are commercially available onthe trademark NORVIR™ (manufactured by Abbott Co.) was used. As KNI-272, a suspension prepared from a fine powder of KNI-272 was used. Groups of dogs (weight: about 10 kg or less), each consisting of three dogs, were used as test animals. Both active compounds were orally administered. In addition, each compound was intravenously administered to control groups to evaluate the dynamics in vivo. The measurement of the change in the non-binding drug concentration in plasma over time was carried out according to a conventional method using blood samples collected from the test animals. Various indexes of dynamics in vivo were also measured.

First of all, the effect of combined use of KNI-272 with Ritonavir on the dynamics in vivo was investigated. It was confirmed that the combined use of KNI-272 created no apparent influence on the dynamics in vivo of Ritonavir within the range of error cause by deviations between individual animals. Specifically, in the observation of the dynamics in viva of Ritonavir with combined KNI-272 which was administered immediately after Ritonavir, there was no significant differences exceeding the differences caused by the deviations between individual animals, although there was a tendency to the MRT value which is an index of the half-life of concentration in blood to be slightly extended when average values are compared with the case where no KNI-272 was administered.

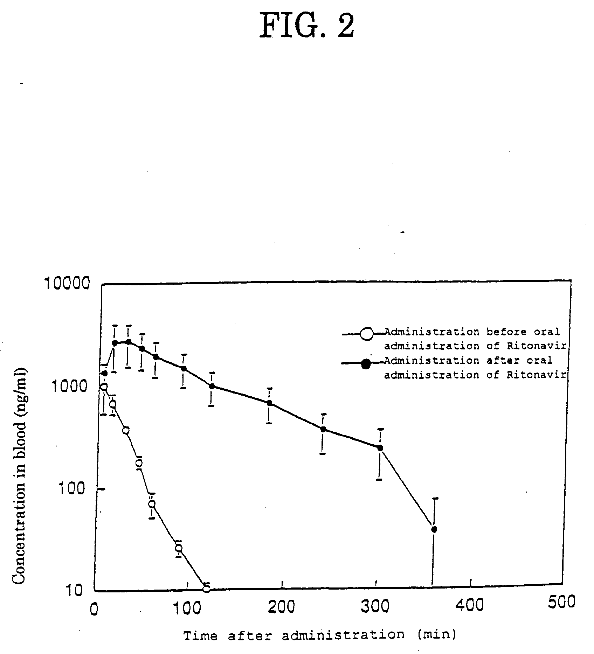

There was a marked difference on the effect of the dynamics in vivo of KNI-272 caused by the combined use of Ritonavir between the case in which Ritonavir was administered before KNI-272 and the case in which Ritonavir was administered after KNI-272. In the case in which Ritonavir was administered after KNI-272, quite naturally the effect on the dynamics in vivo was identical to the case in which Ritonavir was not used within the range of error. On the other hand, when Ritonavir was administered in advance, not only the MRT value which is an index of the half-life of concentration in blood of KNI-272 was remarkably extended, but also there was a significant increase in the concentration maximum value Cmax in plasma. These two effects contributed in cooperation to a synergistic increase in the AUC value which is an index of the rate of oral absorptivity. As one example, the dynamics in vivo of KNI-272 when 15 mg/kg of KNI-272 and one capsule (100 mg/body) of Ritonavir were orally administered at an interval of 30 minutes is shown in Figure 2. In addition, various indexes of dynamics in vivo calculated from the results shown in Figure 2 are given in Table 3. The results obtained from independent intravenous administration of KNI-272 are also given in Table 3.

**TABLE 3**

| Effect of Ritonavir on the metabolism of KNI-272 administration route | | | |
|---|---|---|---|
| | i.v. | p.o.(before)* | p.o.(after)** |
| Dosage (mg/kg) | 15 | 15 | 15 |
| AUC (µg/ml.min) | 605 | 28 | 334 |
| F (%) | - | 4.6 | 55.2 |
| Cₘₐₓ (µg/ml) | - | 1.0 | 2.8 |
| Tₘₐₓ (min) | - | 5 | 30 |
| MRT (min) | 28.2 | 30.8 | 96.8 |
| Clₜₒₜ (l/hr/kg) | 1.49 | - | - |
| V_{d(ss)} (l/kg) | 0.70 | - | - |

| | | | |
|---|---|---|---|
| * KNI-272 was administered before administration of Ritonavir. | | | |
| * * KNI-272 was administered after administration of Ritonavir. | | | |

As can be seen from these results, combined use of Ritonavir and KNI-272 ensures a synergistic extension of the half-life of concentration in blood and increase in the rate of oral absorptivity. In particular, to have best utilization of the advantage of KNI-272 in oral absorptivity it is desirable to administer Ritonavir just before the administration of KNI-272. In addition, this synergistic effect is most remarkable when the amount of Ritonavir administered is set at 10 mg/kg.

### Example 3

An increase in the synergistic anti-virus activity in the combined use of KNI-272 and Ritonavir is shown by a specific experimental example. The anti-virus activity was evaluated as the capability of controlling in vitro generation of infectious virus particles. The effect of controlling cell extinction due to HIV infection was calculated when Ritonavir and KNI-272 were added individually and at the same time to a culture liquid, to determine the concentration region where a synergistic increase in the anti-virus activity in the combined use of the two compounds is conspicuous. Measurement of the in vitro anti-virus activity was carried out according to the following procedure.

### Measurement of anti-HIV activity in vitro

A IIIB strain of HIV-1 was used as an HIV wild strain and CEM-SS cells were used as infected cells in the above-mentioned culture. The amount of HIV sufficient to kill all CEM-SS cells 6 days after inoculation (multiplicy of infection, MOI of 0.01-0.05) was determined and this amount of HIV was added. A 96 well micro-titre plate was used to inoculate 5x10³ CEM-SS cells per well. At the time of inoculation of the cells, a test compound was added to the culture medium to a prescribed concentration. Three specimens were used for each test level. The following controls were used (1) as colorimetric controls: a sample with only a test compound added to the culture medium, (2) as a cytotoxic control for a test compound: a sample with only cells and the test compound added to the culture medium, (3) as a virus control: a sample with the cells inoculated with only a virus, and (4) as a cell survival control, a sample consisting only of cells. In addition, AZT and DDC which are compounds with a known anti-HIV activity were used as positive controls. After the cells were added they were inoculated with the infecting virus, followed by incubation at 37°C for 6 days. Cellular denaturalization due to HIV infection was colorimetrically measured for each well. Employing the colorimetry, was the of metabolism from XTT Tetrazolium to XTT Formazan was evaluated. This colorimetry is called the XTT method (see Weislow et al., Journal of the National Cancer Institute 81, 577-586 (1989)). After the above-mentioned incubation, a prescribed amount of XTT was added to each well and incubation was continued for a further four hours at 37°C to measure the amount of XTT Formazan produced by metabolism by means of colorimetric quantitation.

The anti-HIV activity for each experimental level was calculated by comparing the number of undenatured cells measured by the above-mentioned assay with the corresponding number of cells in the four control groups. Specifically, the proportion of cellular denaturalization due to HIV infection controlled by the addition of the test compound was calculated. A systematic fluctuation between the several experiments was corrected based on the results of AZT and ddC used as positive controls.

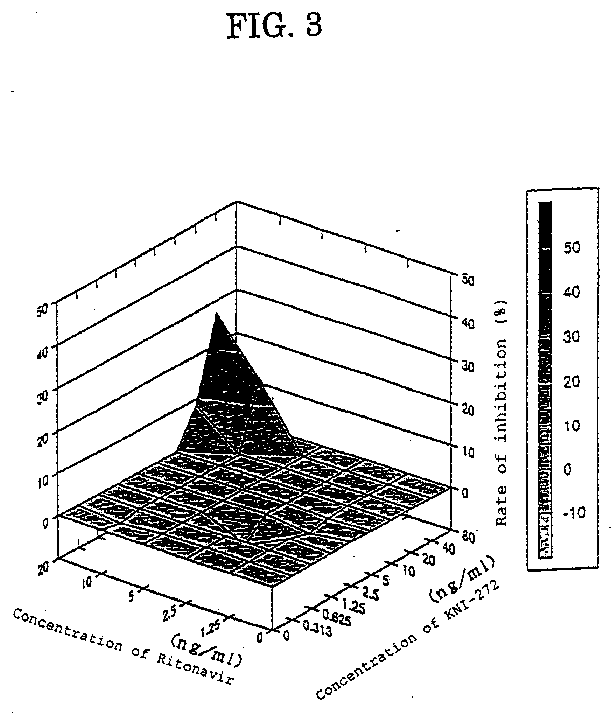

In this example, 0 ng/ml and several concentrations in the range of 1.25-20.0 ng/ml of Ritonavir and 0 ng/ml and several concentrations in the range of 0.313-80.0 ng/ml of KNI-272 were used. The results (anti-HIV activities) obtained under these conditions using either one of these medicaments or both medicaments in combinations are shown in Table 4. Table 5 shows the results calculated by subtracting the sum of anti-HIV activity values at given concentrations of the two medicaments from the anti-HIV activity value obtained by the combined use of the two medicaments at the same concentration. The concentration region in which this difference shows a positive value is judged to exhibit a synergistic increase in the anti-HIV activity. The results of Table 5 are diagrammatically shown in Figure 3.

**TABLE 4**

| KNI-272 (ng/ml) | Anti-HIV activity (%) | | | | | |
|---|---|---|---|---|---|---|
| | Ritonavir (ng/ml) | | | | | |
| | 0 | 1.25 | 2.5 | 5.0 | 10.0 | 20.0 |
| 0 | 0 | 2.0 | 2.3 | 0.5 | 8.9 | 9.5 |
| 0.313 | 5.6 | 6.2 | 4.1 | 9.0 | 5.1 | 13.3 |
| 0.625 | 2.1 | 1.7 | 2.7 | 6.8 | 9.6 | 11.0 |
| 1.25 | 3.8 | 4.2 | 1.3 | 6.9 | 10.1 | 11.1 |
| 2.5 | 2.2 | 1.0 | 1.0 | 4.9 | 4.0 | 16.1 |
| 5.0 | 0.0 | 0.0 | 0.0 | 0.0 | 4.9 | 7.5 |
| 10.0 | 2.5 | 0.0 | 1.4 | 6.8 | 7.7 | 31.6 |
| 20.0 | 12.8 | 22.2 | 20.8 | 15.3 | 23.9 | 36.1 |
| 40.0 | 42.8 | 43.3 | 39.6 | 53.4 | 68.7 | 89.8 |
| 80.0 | 100.0 | 91.6 | 97.3 | 95.8 | 100.0 | 100.0 |

**TABLE 5**

| KNI-272 (ng/ml) | Increase in anti-HIV activity (%) due to combined use | | | | |
|---|---|---|---|---|---|
| | Ritonavir (ng/ml) | | | | |
| | 1.25 | 2.5 | 5.0 | 10.0 | 20.0 |
| 0.313 | -1.4 | -3.8 | 2.9 | -9.4 | -1.8 |
| 0.625 | -2.4 | -1.7 | 4.2 | -1.4 | -0.6 |
| 1.25 | -1.6 | -4.8 | 2.6 | -2.6 | -2.2 |
| 2.5 | -3.2 | -3.5 | 2.2 | -7.1 | 4.4 |
| 5.0 | -2.0 | -2.3 | -0.5 | -4.0 | -2.0 |
| 10.0 | -4.5 | -3.4 | 3.8 | -3.7 | 19.6 |
| 20.0 | 7.4 | 5.7 | 2.0 | 2.2 | 13.8 |
| 40.0 | -1.5 | -5.5 | 10.1 | 17.0 | 37.5 |

Under the above-mentioned experimental conditions used in this example, it was seemingly found that there was no synergistic increase in the antivirus activity the range wherein a simple arithmetic sum of the control rate achieved by independent addition of these compounds exceeds 95%. With regard to this concentration region wherein no synergistic increase can be seemingly identified, a similar evaluation was carried out adding a larger amount of HIV to qualitatively evaluate the presence or absence of a synergistic effect. Because the amount of HIV added is too high for the virus control to exhibit a true value, non-infected cells (cells which are not denaturalized) in the virus control are exhausted within a much shorter period than six days. This affords qualitative results rather than quantitative results.

An can be understood from the results shown in Table 4 and Table 5, when Ritonavir was added in the amount of 20.0 ng/ml, a synergistic increase was seen with the addition of KNI-272 in an amount of 10.0-40.0 ng/ml. When the amount of Ritonavir was 10.0-20.0 ng/ml, a clear synergistic increase was seen when 20.0-40.0 ng/ml of KNI-272 was added. The results of synergistic increase obtained by the addition of 80.0 ng/ml of KNI-272 appeared. However, another experiments using 80.0 ng/ml of KNI-272, but adding a larger amount of HIV, confirmed a synergistic effect comparable to the case in which 20.0 ng/ml of KNI-272 was used, although the result was qualitative. Similarly, when Ritonavir was added in the amount of 80.0 ng/ml, a synergistic increase was confirmed qualitatively when 10.0-40.0 ng/ml of KNI-272 was used.

These results clearly confirm a synergistic increase in the anti-HIV activity by combined use of Ritonavir and KNI-272. This is more clearly understood from the total sum of the volume of the part shaded by oblique lines in Figure 3. That is, the calculated value of 20 µM²% or larger in this chart is within the range of 0 to 50 µM²% which is considered to be an index representing a remarkable synergistic increase in anti-HIV activity (Antimicrobial Agents and Chemotheraphy, 39, 2718-2727 (1995) etc. reference), proving a manifest synergistic increase resulting from the combined use of the two medicaments. Comparing these results with the case where these medicaments are individually used, an anti-HIV activity equivalent to that obtained when 20.0 ng/ml of Ritonavir is independently added (about 10% anti-HIV activity) can be obtained by using about 40.0 ng/ml of KNI-272. It is surprising that addition of KNI-272 in the amount of 60.0 ng/ml, which is equivalent to the total sum of 20.0+40.0 ng/ml, exhibits only about 3/4 of the anti-HIV activity that exhibited by the combined use of 20.0 ng/ml of Ritonavir and 40.0 ng/ml of KNI-272.

Although it is true that the combined use of Ritonavir and KNI-272 brings about a synergistic increase in anti-HIV activity in vitro in a broad concentration range, in order for a manifest AIDS treatment effect to be achieved by using only essential active components exhibiting HIV protease inhibitive action, a practical concentration range should be selected from the range in which about 50% or more anti-HIV activity can be achieved. Taking this factor into consideration, useful concentration ranges are as follows. Specifically, when Ritonavir is added in an amount of 20.0-40.0 ng/ml, the amount of KNI-272 is selected from the range of 30.0-80.0 ng/ml; and when Ritonavir is added in an amount of 10.0-20.0 ng/ml, the amount of KNI-272 is selected from the range of 40.0-80.0 ng/ml. More preferably, if a synergistic effect providing an anti-virus activity in vitro exceeding about 70% is desired, the concentration region to be selected should be as follows. That is, when Ritonavir is added in an amount of 20.0-40.0 ng/ml, the amount of KNI-272 is selected from the range 40.0-80.0 ng/ml; when Ritonavir is added in an amount of 10.0-20.0 ng/ml, the amount of KNI-272 is 60.0 ng/ml or more.

The above concentration range can be generalized by the product of the concentrations of Ritonavir and KNI-272. Specifically, when the amounts of these components to be added are limited to the ranges of 20.0-40.0 ng/ml and 20.0-80.0 ng/ml, respectively, the product, [Ritonavir]x[KNI-272] should be more than 800 (ng/ml)², and more preferably the product [Ritonavir]x[KNI-272] is selected from the range exceeding 1200 (ng/ml)². Alternatively, defining the added amount in terms of the concentration ratio of the two compounds, when the amount of Ritonavir to be added is limited to the range of 20.0-40.0 ng/ml and that of KNI-272 to 10.0-80.0ng/ml, the concentration ratio [Ritonavir]:[KNI-272] should be 1:4 to 2:1; and particularly when the amounts of Ritonavir and KNI-272 to be added are limited to the range of 20.0-40.0 ng/ml and 20.0-80.0 ng/ml, respectively, the concentration ratio [Ritonavir]:[KNI-272] should be in the range of 1:2-2:1.

It is unnecessary to mention that when a lower anti-HIV activity is acceptable, such as a case where the purpose of administration is to prevent AIDS symptoms from being manifested, a smaller value of the product [Ritonavir]x[KNI-272] can be applied. A lower anti-virus activity induced by HIV protease inhibitive action can be used also in the a three-component drug composition in which a reverse transcription enzyme inhibitor is incorporated in addition to the essential active components exhibiting the HIV protease inhibitive action. Specifically, when the amounts of Ritonavir and KNI-272 are limited to the ranges of 20.0-40.0 ng/ml and 10.0-80.0 ng/ml respectively, the product [Ritonavir]x[KNI-272] larger than 600 (ng/ml)², particularly larger than 800 (ng/ml)², is an ideal range for the three-component composition containing a reverse transcription enzyme inhibitor in addition to the essential active components exhibiting the HIV protease inhibitive action, in view of the fact that anti-virus activity exceeding 50% as well as a synergistic increase of the effects are exhibited.

### Example 4

A synergistic extension of the half-life of concentration in blood and increase in the rate of oral absorptivity achieved by the embodiment, which is parallel administration of KNI-272 and Indinavir, in the AIDS therapeutic agent of the present invention will be described by way of a specific experimental example. In this experiment, the half-life of concentration in blood and the rate of oral absorptivity of KNI-272 and Indinavir, when these medicaments are individually administered, were first compared. It was confirmed that the half-life of concentration in blood of KNI-272 is much lower than that of Indinavir. Taking this into consideration, major attention was given to the change in concentration in blood of KNI-272 over time to evaluate the presence or absence of a synergistic extension of the half-life of concentration in blood or a synergistic increase in the rate of oral absorptivity by the combined use of these compounds.

The following drug interactions are generally given as factors causing fluctuations in dynamics in vivo:
(1) interaction relating to drug absorption,
(2) interaction relating to plasma protein binding,
(3) interaction induced by a transporting carrier when passing through a biological membrane (such as intake into liver or kidney, bile, excretion to urine), and
(4) interaction occurring during a metabolism process.

Among these, the interaction (1) causes a decrease in the rate of oral absorptivity during oral administration, the interaction (2) has almost no effect on the concentration in plasma, typically the concentration of non-binding type medicines. The interactions (3) and (4) are not affected by the manner of administration, but exhibit some effect on the drug interaction in fluctuation of the dynamics in vivo. On the other hand, the factor having a significant effect on fluctuation of dynamics in vivo during combined use such as an increase in the rate of oral absorptivity, superficially, an increase in a peak concentration in blood, is the interaction (1).

In this example, the change in concentration in blood of KNI-272 over time when the KNI-272 was orally administered in combination with Indinavir, i.e. the change in the concentration of a non-binding type of drug in plasma over time, was measured to evaluate the effect of drug interactions on fluctuations of dynamics in vivo. A commercially available Indinavir for oral administration, CRIXIVAN™ (manufactured by Merck), was used after dissolving Indinavir sulfate in a prescribed concentration. As KNI-272, a suspension prepared from a fine powder of KNI-272 was used. Rats were used as test animals. Both active compounds were orally administered. In addition, each compound was individually administered to control groups to evaluate the dynamics in vivo. The animals were fasted for 16 hours before administration. The measurement of the change in the non-binding drug concentration in plasma over time was carried out according to a conventional method using blood samples collected from the test animals.

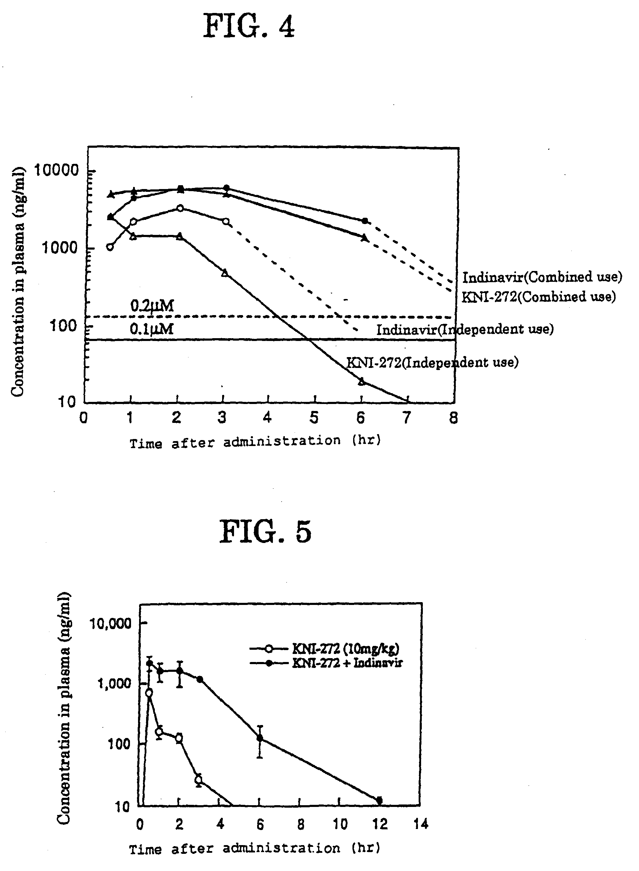

First, the effect of the combined use of KNI-272 with Indinavir on dynamics in vivo was investigated. As shown in Figure 4, an increase concentration in the plasma of Indinavir was seen when the two medicaments were administered at the same time. The degree of increase was much more than the range of error which may produced by deviations between individual animals. The increase was as much as twice in terms of maximum concentration in blood value (Cmax). In some extension of the half-life of concentration in blood observed. These have synergistically increased the value of AUC (the area below the plasma concentration curve) which is an index for the rate of oral absorptivity. Specifically, in respect of Indinavir an increase in the AUC value from 17,200 ng/ml.hr to 44,300 ng/ml.hr (almost two times) was achieved by the adminstration conditions of Figure 4 under which 40 mg/kg of KNI-272 was administered together with 40 mg/kg of Indinavir. Especially, an increase in the concentration of Indinavir in plasma during the six hours after administration is conspicuous. On the other hand, an increase of about twice was also seen in the maximum concentration value in blood (Cmax) of KNI-272. A considerable extension of the half-life of concentration in blood was also seen. Under the conditions of Figure 4 the half-lives of concentration in blood of KNI-272 and Indinavir were almost the same. Reflecting the conspicuous rise in the maximum concentration value in blood (Cmax) and extension of the half-life of concentration in blood, KNI-272 exhibited about a six-times increase in the value of AUC, from 4,900 ng/ml.hr to 29,200 ng/ml.hr. Especially, an increase in the concentration of KNI-272 in plasma during the six hours after administration is conspicuous.

The effect on the movement in vivo of KNI-272 brought about by the combined use of Indinavir is believed to be due to the above-mentioned interaction (3) or (4). To confirm this supposition, a change in the plasma concentration of KNI-272 over time was investigated for the case in which Indinavir was administered in advance. As shown in Figure 5, when KNI-272 was administered one hour after the administration of Indinavir, KNI-272 exhibited as much as a 3-times increase in the maximum concentration value in blood (Cmax). The half-life of concentration in blood also increased. Under the conditions of Figure 5, i.e., by the administration of 90 mg/kg of Indinavir one hour before the administration of 10 mg/kg of KNI-272, the AUC value of KNI-272 increased about 10 times from 660 ng/ml.hr to 6,900 ng/ml.hr, reflecting an increase in the maximum concentration value in blood (Cmax) and significant extension of the half-life of concentration in blood. In particular, with regard to the increase in the concentration of KNI-272 in plasma during six hours after the administration, the degree of this increase in the case where Indinavir was previously administered was more conspicuous than in the case of simultaneous administration. A further delay of the administration of KNI-272, that is, administration of KNI-272 at two hours after Indinavir administration, revealed a result similar to that shown in Figure 5. Administration of KNI-272 after three hours, at which the concentration of Indinavir in plasma begins to decrease, demonstrated some effect of combined use, but this effect was little.

These results show that combined use of Indinavir and KNI-272 achieves a synergistical extension of the half-life of the concentration in blood and an increase in rate of oral absorption. In particular, to make best use of the advantage of the increase in the rate of oral absorptivity of KNI-272, previous administration of Indinavir is recommended. Specifically, when the dosage of Indinavir is higher than the dosage of KNI-272, for example, when the dosage ratio (weight) of KNI-272 and Indinavir is about 9:1, KNI-272 should be administered within two hours after administration of Indinavir. On the contrary, when the dosage of Indinavir is lower than or the same as the dosage of KNI-272, for example, when the dosage ratio (weight) of KNI-272 and Indinavir is about 1:2, simultaneous administration, which is suitable for realizing an increase in the maximum concentration value in blood Cmax of Indinavir, is applicable.

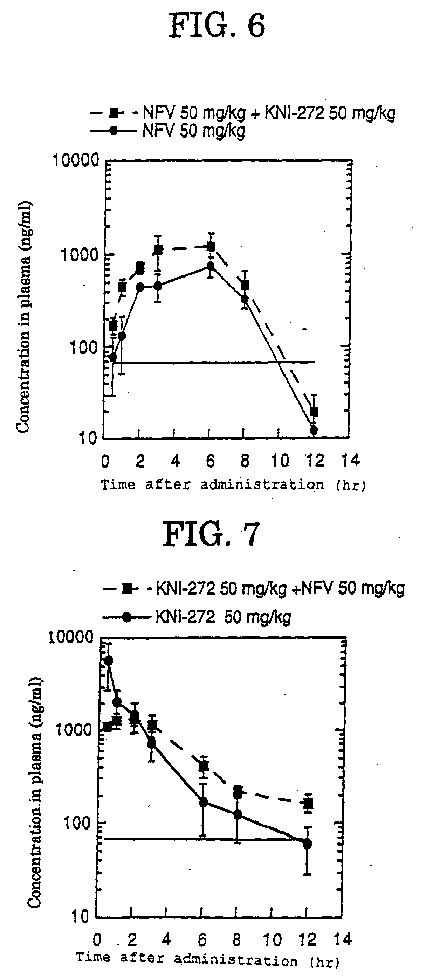

A synergistic extension of the half-life of concentration in blood and an increase in the rate of oral absorptivity achieved by the combined use of Nelfinavir and KNI-272 was investigated in the same manner. The method of evaluation was applied to the above-mentioned system of Indinavir and KNI-272 was followed. The dynamics in vivo, specifically, change in the concentration in blood over time exhibited by independednt oral administration of Nelfinavir is very similar to that of Indinavir. The synergistic extension of the half-life of concentration in blood and increase in the rate of oral absorptivity by the combined use of Nelfinavir and KNI-272 also resembled the case of the combined use of Indinavir and KNI-272. The results of the experiments in which both compounds were orally administered at the same time are shown in Figure 6 and Figure 7. In this example, a dosage of 50 mg/kg was selected for both Nelfinavir and KNI-272. A remakable extension in the half-life of concentration in blood and increase the maximum concentration value in blood (Cmax) due to the combined use were seen in KNI-272.

The increase concentration in the plasma of KNI-272 during six hours after administration was particularly conspicuous. Nelfinavir also exhibited a clear increase in the concentration in blood as demonstrated by the increase in the maximum concentration value in blood (Cmax) after six hours. Reflecting these effects, the AUC value for KNI-272 exhibited a slight increase from 550 µg/ml.min to 620 µg/ml.min, in spite of a significant decrease in the maximum concentration value in blood Cmax to about 1/4. The AUC value for Nelfinavir exhibited about a 1.6 times increase, from 130 µg/ml.min to 215 µg/ml.min, in proportion to the increase in the maximum concentration value in blood (Cmax). In particular, to make best use of the advantage of the increase in the rate of oral absorptivity of KNI-272, previous administration of Nelfinavir is recommended. Simultaneous administration is recommended to increase the maximum concentration value in blood (Cmax) of Nelfinavir. With regard to selection of the administration timing, the above-mentioned relationship in the composition comprising Indinavir and KNI-272 is exactly applicable.

Although rats were used as test animals in this example, the effects on concentration in bloods obtained by the combined use can be reproduced in a human being substantially in the same manner. The above-mentioned conditions of administration timing and the dosage ratio can be applied to human beings, provided the ratio of body weight and volume of blood, or the ratio of body weight and blood vessel surface area are appropriately adjusted to determine the dosage by which the desired concentration in bloods can be achieved.

### Example 5

An increase in the synergistic anti-virus activity in the combined use of KNI-272 and Indinavir is shown by a specific experimental example. The anti-virus activity was evaluated as the capability of controlling in vitro generation of infectious virus particles. The effect of controlling cell extinction due to HIV infection was calculated when Indinavir and KNI-272 were added individually and at the same time to a culture liquid, to determine the concentration region where a synergistic increase in the anti-virus activity in the combined use of the two compounds is conspicuous. Measurement of the in vitro anti-virus activity was carried out according to the following process.

### Measurement of anti-HIV activity in vitro

A IIIB strain of HIV-1 was used as an HIV wild strain and CEM-SS cells were used as infected cells in the above-mentioned culture. The amount of HIV sufficient to kill all the CEM-SS cells 6 days after inoculation (multiplicy of infection, MOI of 0.01-0.05) was determined and this amount of HIV was added. A 96 well micro-titre plate was used to add 5x10³ CEM-SS cells per each well. At the time of addition of cells, a test compound was added to the culture medium to a prescribed concentration. Three specimens were used for each test level. The following controls were used (1) as colorimetric controls: a sample with only a test compound added to the culture medium, (2) as a cytotoxic control for a test compound: a sample with only cells and the test compound added to the culture medium, (3) as a virus control: a sample with the cells inoculated with only a virus, and (4) as a cell survival control: a sample consisting only of cells. In addition, AZT and DDC which are compounds with a known anti-HIV activity were used as positive controls. After the cells were added, they were with the infecting virus, followed by incubation at 37°C for 6 days. Cellular denaturalization due to HIV infection was colorimetrically measured for each well. Employing the colorimetry, was the of metabolism from XTT Tetrazolium to XTT Formazan was evaluated. This colorimetry is called the XTT method (see Weislow et al., Journal of the National Cancer Institute 81, 577-586 (1989)). After the above-mentioned incubation, a prescribed amount of XTT was added to each well and incubation was continued for a further four hours at 37°C to measure the amount of XTT Formazan produced by metabolism by means of colorimetric quantitation.

The anti-HIV activity for each experimental level was calculated by comparing the number of undenatured cells measured by the above-mentioned assay with the corresponding number of cells in the four control groups. Specifically, the proportion of cellular denaturalization due to HIV infection controlled by the addition of the test compound was calculated.

In this example, 0 ng/ml and several concentrations in the range of 12.5-50.0 ng/ml of Indinavir and 0 ng/ml and several concentrations in the range of 20.0-80.0 ng/ml of KNI-272 were used. The results obtained under these conditions using either one of these medicaments or both medicaments in combinations are used to calculate a concentration range in which a synergistic effect is exhibited by the combined use of the two medicaments. In the results shown in Table 6, the concentration region in which the value is positive is considered to exhibit a synergistic increase in anti-HIV activity.

**TABLE 6**

| Dose of KNI-272 (ng/ml) | Increase in anti-HIV activity(%) | | | |
|---|---|---|---|---|
| | Dose of Indinavir(ng/ml) | | | |
| | 0 | 12.5 | 25.0 | 50.0 |
| 0 | | | | - |
| 20.0 | | | - | - |
| 40.0 | | - | - | 2.4 |
| 80.0 | - | - | 5.0 | 9.5 |

In the results shown in the above table, only those concentration ranges with a positive numerical value exhibited an increase in the anti-HIV activity in statistical meaning. Since the anti-virus activity itself exceeded 80% in these concentration ranges, the synergistic effect was considerably a little in appearance. Estimating from these results, a synergistic effect was exhibited, even if only to a very small degree, when the concentration range of the added medicaments, in terms of the product [Indinavir]x[KNI-272], was more than 1600 (ng/ml)², particularly more than 3200 (ng/ml)2. Within this range of concentrations, it is more preferably to obtain good results to select a ratio of concentrations of added medicaments, [Indinavir]:[KNI-272], in the range from 1:4 to 2:1, particularly from 1:2 to 2:1.

### Example 6

Indinavir and KNI-272 were proven to exhibit an anti-virus activity against drug resistant strain viruses of each other to the same degree as these medicaments exhibit to their respective wild strain viruses. A drug resistant strain virus is a resistance strains induced in a cell culture system in vitro while maintaining infection in the presence of each medicament through generations. The results of one example are shown in Table 7. As can be seen from the results shown in this Table, the two compounds do not exhibit a cross resistance, indicating that KNI-272 is effective against Indinavir resistant strains and Indinavir is effective against KNI-272 resistant strains.

**TABLE 7**

| HIV-virus strain | Anti-virus activity IC₅₀ (µg/ml) | |
|---|---|---|
| | Indinavir | KNI-272 |
| Wild strain (IIIB) | 0.009 | 0.007 |
| Indinavir resistant strain | 0.576 | 0.010 |
| KNI-272 resistant strain | 0.030 | 0.320 |

Similar to Indinavir, Nelfinavir was proven to exhibit an anti-virus activity against virus strains resistant to KNI-272 to the same degree as Nelfinavir exhibits against wild strains. The results of one example are shown in Table 8. As shown in this Table, Nelfinavir exhibits an anti-virus activity against virus strains resistant to KNI-272 of the same degree as its exhibits against wild strains. Nelfinavir also exhibits a high anti-virus activity against a variant strain which has acquired drug resistance against Ritonavir which is a commercially available anti-AIDS medicine. On the other hand, KNI-272 is not effective against this Ritonavir resistant strain. From these results, it can be concluded that the two compounds, Nelfinavir and KNI-272, do not exhibit a cross resistance, indicating that KNI-272 is effective against Nelfinavir resistant strains and Nelfinavir would be effective against KNI-272 resistant strains.

**TABLE 8**

| HIV-virus strain | Anti-virus activity IC₅₀ (µg/ml) | |
|---|---|---|
| | Nelfinavir | KNI-272 |
| Wild strain (IIIB) | 0.007 | 0.007 |
| Ritonavir resistant strain | 0.124 | 0.304 |
| KNI-272 resistant strain | 0.030 | 0.320 |

### Example 7

### 〈Formulation 1〉

A peroral drug composition (Agent A) which contains only KNI-272 as an active component was prepared in accordance with the method described in Japanese Patent Application Laid-open No. 208478/1996. Specifically, granules which comprises an average composition shown in the following Table 9 was prepared and filled into capsules made of hard gelatin. The content of the active component KNI-272 was 150 mg per capsule.

**TABLE 9**

| Average composition | |
|---|---|
| KNI-272 | 50 g |
| Hydroxypropylmethylcellulose 2910 | 20 g |
| Nucleic particles: globular crystalline cellulose (Cellfire CP203, manufactured by Asahi Chemical Industries, Inc.) | 500 g |

A peroral drug composition (Agent B) which contains only Saquinavir methanesulfonate as an active component was prepared using a commercially available hard gelatin capsule (INVIRASE™, manufactured by Roche Company). Each capsule contained 200 mg of the active component as Saquinavir. The formulation comprising Saquinavir and KNI-272 in combination, which is one embodiment of the present invention, consisted of 1-4 capsules of Agent A and 1-4 capsules of Agent B per dose. This dosage is administered to a male adult usually every 8 hours, three times a day.

### (Formulation 2)

The above-mentioned Agent A prepared in the Formulation 1 which contains 150 mg/capsule of KNI-272 as a sole active component was used as A for oral administration.

Hard gelatin capsules commercially available on the trademark NORVIR (Abbott Co.) were used as an agent containing only Ritonavir as an active component for oral administration (Agent C). Each capsule contained 100 mg of the active component as Ritonavir.

The formulation comprising Ritonavir and KNI-272 in combination, which is another embodiment of the present invention, consisted of four capsules, two Agent A capsules and two Agent B capsules, usually administered to a male adult every 8 hours, three times a day.

### (Formulation 3)

The above-mentioned Agent A prepared in the Formulation 1 which contains 150 mg/capsule of KNI-272 as a sole active component was used as A for oral administration.

Capsules for oral administration containing only Indinavir as an active component, which are commercially available on the trademark CRIXIVAN (Merck Co.), were used (Agent D). Each capsule contained 200 mg of the active component as Indinavir. In addition, an agent for oral administration which contains only Nelfinavir as an active component commercially available tablets, VIRACEPT™ (Agouron Co.), were used as Agent E. One tablet of Agent E contained 250 mg of Nelfinavir as an active component.

The formulation comprising Indinavir and KNI-272 in combination, which is a further embodiment of the present invention, consisted of four capsules, two Agent A capsules and two Agent B capsules. The formulation comprising Nelfinavir and KNI-272 in combination, which is a furthermore embodiment of the present invention, consisted of four capsules, two Agent A capsules and two Agent E capsules. Each formulation was designed to be administered to a male adult every 8 hours, three times a day.

### INDUSTRIAL APPLICABILITY

The AIDS therapeutic agent of the present invention comprises two medicaments as essential active components, each medicament capable of inhibiting the enzyme activity of asparatic protease originating from a human immunodeficiency virus which causes acquired immune deficiency syndrome (AIDS). Combinations of the two medicaments are KNI-272 and Saquinavir, KNI-272 and Ritonavir, KNI-272 and Indinavir, and KNI-272 and Nelfinavir. The dosage and proportion which bring about a synergistic treatment effect of combined use of these medicaments are selected. Specifically, a synergistic increase in the anti-virus activity, a synergistic increase the maximum concentration value in blood, or a synergistic extension of the half-time of concentration in blood can be achieved. These synergistic effects ensure a relatively smaller dosage of the active components to achieve a treatment effect equivalent to or higher than the effect achieved by individual administration of either one of the medicaments which have conventionally been used. In addition, a decrease in the relative dosage of an individual medicament makes it possible to significantly mitigate undesirable side effects caused by a high dosage of each medicament. Furthermore, with regard to practical application of the AIDS therapeutic composition of the present invention, extension of the time during which the concentration in bloods of the two medicaments are maintained in an effective concentration range and prolongation of administration intervals provide the great

## Claims

1. An AIDS therapeutic composition comprising, as essential effective components which bring about a human immunodeficiency virus protease inhibition action, (R)-N-(1,1-dimethylethyl)-3-[(2S,3S)-2-hydroxy-3-[(R)-2-(5-isoquinolyloxyacetyl)amino-3-methylthiopropanoyl] amino-4-phenylbutanoyl]-1,3-thiazolidine-4-carboxyamide (KNI-272) and a compound selected from the group consisting of N-(1,1-dimethylethyl)-decahydro-2-[2(R)-hydroxy-4-phenyl-3(S)-{[N-(2-quinolylcarbonyl)-L-asparaginyl]amino}butyl]-(4aS, 8aS)-isoquinoline-3(S)-carboxyamide (Saquinavir), 5S-(5R*, 8R*, 10R*,11R*)-10-hydroxy-2-methyl-5-(1-methylethyl)-1-[2-(1-methylethyl)-4-thiazolyl]-3,6-dioxo-8,11-bis(phenyl methyl)-2,4,7,12-tetrazatridecane-13-hydroxy acid, 5-thiazolylmethyl ester (Ritonavir), [1(1S, 2R),5(S)]-2,3,5-trideoxy-N-(2,3-dihydro-2-hydroxy-1H-inden-1-yl)-5-[2-{[(1,1-dimethylethyl)amino]carbonyl}-4-(3-pyridinylmethyl)-1-piperazinyl]-2-(phenylmethyl)-D-erythropentonamide (Indinavir), and [3S-2(2S*, 3S*), 3α, 4aβ, 8aβ ]]-N-(1,1-dimethylethyl)-decahydro-2-[2-hydroxy-3-(3-hydroxy-2-methylbenzoyl)amino]-4-(phenylthio)butyl]-3-isoquinolinecarboxamide (Nelfinavir), in an amount and proportion in which a synergistic treatment effect can be exhibited.

2. The AIDS therapeutic composition according to claim 1, having a dosage form which enables KNI-272 and one compound selected from the group consisting of Saquinavir, Ritonavir, Indinavir, and Nelfinavir to be orally administered simultaneously.

3. The AIDS therapeutic composition according to claim 1, having a dosage form which enables one compound selected from the group consisting of Saquinavir, Ritonavir, Indinavir, and Nelfinavir to be orally administered first followed by the oral administration of KNI-272.

4. The AIDS therapeutic composition according to any one of claims 1 to 3, having a dosage form which enables 5-25 mg/kg (patient's body weight, hereinafter the same) of Indinavir and 3-15 mg/kg of KNI-272 to be administered.

5. The AIDS therapeutic composition according to any one of claims 1 to 3, having a dosage form which enables 3-15 mg/kg of Nelfinavir and 3-15 mg/kg of KNI-272 to be administered.

6. The AIDS therapeutic composition according to any one of claims 1 to 3, having a dosage form which enables 3-25 mg/kg of Saquinavir and 3-15 mg/kg of KNI-272 to be administered.

7. The AIDS therapeutic composition according to any one of claims 1 to 3, having a dosage form which enables 3-15 mg/kg of Ritonavir and 3-15 mg/kg of KNI-272 to be administered.
